# EUROPÄISCHE PATENTANMELDUNG

(11) **EP 4 652 973 A1**
(43) Veröffentlichungstag der Anmeldung: **26.11.2025**
(21) Anmeldenummer: 25175616.9
(22) Anmeldetag: 12.05.2025
(51) Int. Cl.: A61F 5/01, A61F 5/14

(54) **ORTHESE ZUR BEHANDLUNG, ZUR KORREKTUR UND/ODER ZUR VORBEUGUNG VON FUSSFEHLSTELLUNGEN**

(30) Priorität: 23.05.2024 DE 202024102657 U
(71) Anmelder: FXF GmbH, 92224 Amberg (DE)
(72) Erfinder: FISCHER, Franz, 92224 Amberg (DE)
(74) Vertreter: Benninger, Johannes

(57) **Zusammenfassung**

Es ist eine Orthese (10) zur Korrektur und/oder zur Behandlung von Fußfehlstellungen offenbart, die sich insbesondere für eine Behandlung von Hallux-Valgus eignen kann. Die Orthese (10) umfasst einen Sohlenteil (12) zur Aufnahme zumindest eines Teils eines darauf stehenden und sich darauf abstützenden menschlichen Fußes sowie einen für die Aufnahme und Abstützung zumindest einer Unterseite einer Großzehe des auf dem Sohlenteil (12) stehenden Fußes sowie zumindest von medialen Teilbereichen der Großzehe vorgesehenen Vorderteil (14).

Der Vorderteil (14) ist über einen Gelenkabschnitt (16) gelenkig mit dem Sohlenteil (12) verbunden. Der Gelenkabschnitt (16) befindet sich in einem Bereich unterhalb und vorderhalb eines Zehengrundgelenks der Großzehe bei auf dem Sohlenteil (12) stehendem Fuß.

## Beschreibung

Die vorliegende Erfindung betrifft eine Orthese, die der Behandlung, der Korrektur und/oder der Vorbeugung von Fußfehlstellungen dient, insbesondere im Zusammenhang mit dem Beschwerdebild des Hallux Valgus.

Fußfehlstellungen, insbesondere Hallux Valgus, treten krankheits- und/oder altersbedingt und/oder durch länger andauerndes Tragen einer falschen oder ungeeigneten Fußbekleidung bei größeren Teilen der Bevölkerung regelmäßig auf. Bei Hallux Valgus verändert die Großzehe ihre Position und wandert, ausgelöst durch eine Abflachung der inneren Längswölbung am Fuß und einer Außenrotation im Unterschenkel, nach außen ab. Je nach Ausprägung kann Hallux Valgus bei den davon betroffenen Personen zu Gehbinderungen und Schmerzen führen.

Das Krankheitsbild des Hallux Valgus kann mittels Röntgenbildes bildgebend und/oder mittels des sog. Hallux Valgus-Winkels klinisch in verschiedene Ausprägungs- oder Schweregrade eingeteilt werden. Je nach Schweregrad stehen zur Therapie des Hallux Valgus konservative Verfahren, beispielsweise im Rahmen der Physiotherapie sowie der Orthopädietechnik, und/oder operative Verfahren zur Verfügung. Die sinnvolle Auswahl des Verfahrens zur Korrektur der Fehlstellung hängt in erster Linie vom Schweregrad der Fehlstellung des Betroffenen ab. Zu den konservativen Verfahren gehören beispielsweise Schienen, Bandagen, Tapes, Socken mit eingearbeiteten Tapes und Silikonkeile.

Durch DE 201 12 537 U1 und DE 20 2005 006 701 U1 sind jeweils Bandagen für einen Großzeh offenbart, welche jeweils durch Elastikbänder gebildet sind. Eine solche Großzehbandage umfasst zwei Verbindungen und einen Verschluss, wobei das Elastikband an zwei Kreuzungspunkten durch die beiden Verbindungen in einer Weise zusammengehalten wird, dass der Großzeh vom Elastikband derart umgeben ist, dass das Elastikband an der Oberseite des Großzehs entlang über den ersten Kreuzungspunkt am Ansatzpunkt des Großzehs am Fuß und auf der Unterseite des Fußes diagonal nach hinten zu einer Schlaufe um den Kreuzungspunkt herumgeführt wird. Über den Verschluss kann die Bandage am Knöchel des Halters fixiert werden.

Die DE 92 04 651 U1 offenbart eine medizinische Orthese zur Behandlung von Belastungsdeformitäten im Bereich der Großzehe. Die Orthese besteht aus einem elastisch bandförmig ausgebildeten Material zur Korrektur von Hallux Valgus. Am vorderen Ende des Bandes ist ein zirkulär an der Großzehe angebrachter, in seinem Umfang stufenlos einstellbarer Klettverschluss fest befestigt. Mittig an der Außenseite des Bandes ist eine Kletthaftfläche vorgesehen, an der ein Klettband mit einem Ende befestigt ist, wobei das freie um den Mittelfuß legbare Ende des Klettbandes an der Kletthaftfläche befestigt ist. Zudem ist am hinteren Ende des Bandes, innenseitig ein Klettband befestigt, welches um den Knöchel gelegt und an der Kletthaftfläche befestigt ist.

Bei diesen bekannten Lösungen hat es sich jedoch als nachteilig erwiesen, dass die Bandage bei der Anwendung jeweils vom Träger selbst zu wickeln ist und/oder der Wickelvorgang sich als kompliziert erwiesen hat, da die Kreuzungspunkte jeweils an exakt vorgegebenen Stellen anzuordnen sind, um entsprechende Zugkräfte auf die Großzehe bewirken zu können. Bei mangelnder Erfahrung und bei fehlerhafter Wicklung kann folglich das Gegenteil einer Korrektur von Zehfehlstellungen bewirkt werden.

Weiterhin ist aus der DE 10 2021 108 100 A1 ein Schuhwerk mit einem elastischen Korrekturband zur Behandlung und Vorbeugung von Fußfehlstellungen bekannt. Das als leichte Sandale ausgestaltete Schuhwerk weist ein Halteelement zum Fixieren eines Fußes relativ zu dem Schuhwerk und ein an einer Zehe zu befestigendes Spannelement auf. Das Spannelement ist dafür eingerichtet, in einem an dem Fuß befestigten Zustand des Schuhwerks eine erste Korrekturkraft auf die Zehe auszuüben und eine der ersten Korrekturkraft entgegen gerichtete zweite Korrekturkraft auf ein Zehengrundgelenk auszuüben. Das Spannelement ist durch ein elastisches Korrekturband gebildet.

Allerdings hat es sich bei den bekannten Schuhen zur Korrektur der beschriebenen Fuß- oder Zehfehlstellungen als nachteilig erwiesen, dass in der Regel eine individuelle Anpassung an den Fuß des jeweiligen Trägers unumgänglich ist. Hinzu kommt bei den mit Schlaufen ausgestatteten Korrektureinrichtungen, dass sie sich hauptsächlich nur für Sandalen eignen, da sich insbesondere das Anziehen geschlossener Schuhe wegen des notwendigen Einfädelns der Zehen in die bereitgestellten elastischen Schlaufen sehr mühsam gestaltet.

Angesichts der festgestellten Nachteile und Einschränkungen der bekannten Korrektursysteme und Schuhe kann es als vorrangiges Ziel der vorliegenden Erfindung betrachtet werden, eine universell einsetzbare Fuß-Orthese zur Korrektur und/oder zur Vorbeugung von Fußfehlstellungen zur Verfügung zu stellen, die insbesondere im Zusammenhang mit der Behandlung von Hallux valgus einsetzbar ist, und die sich problemlos mit jeglichen Arten von offenen Schuhen wie Sandalen sowie auch von geschlossenen Schuhen kombinieren lässt, ohne dass es zu unzumutbaren Erschwernissen für den Träger beim Tragen der Orthese und/oder eines damit ausgestatteten Schuhs kommt.

Dieses Paket an identifizierten Zielen wird durch den Gegenstand des unabhängigen Anspruchs erreicht. Weitere vorteilhafte Ausgestaltungen werden durch die abhängigen Ansprüche beschrieben.

Zur Erreichung zumindest eines Teils der oben genannten Ziele wird mit der hier beschriebenen Erfindung eine Orthese zur Behandlung, zur Korrektur und/oder zur Vorbeugung von Fußfehlstellungen, insbesondere im Zusammenhang mit Hallux Valgus, vorgeschlagen. Die erfindungsgemäße Orthese umfasst zumindest einen Sohlenteil zur Aufnahme zumindest eines Teils eines darauf stehenden und sich darauf abstützenden menschlichen Fußes sowie einen für die Aufnahme und Abstützung zumindest einer Unterseite einer Großzehe des auf dem Sohlenteil stehenden Fußes sowie zumindest von medialen Teilbereichen der Großzehe vorgesehenen Vorderteil.

Der Vorderteil ist über einen Gelenkabschnitt gelenkig mit dem Sohlenteil verbunden. Der Gelenkabschnitt befindet sich in einem Bereich, der etwas vorderhalb eines Zehengrundgelenks der Großzehe bei auf dem Sohlenteil stehendem Fuß liegt, da sich dort ausreichend Platz für die Unterbringung eines solchen Gelenkabschnittes findet, ohne dass es zu störenden Kontaktierungen mit der Fußunterseite und zu drückenden Stellen am Fuß kommen muss.

Die erfindungsgemäße Orthese eignet sich grundsätzlich zum Tragen als Schuheinlage oder generell als Einlageelement für ein - wie auch immer gestaltetes - Fußoberbekleidungsstück, d.h. einen Schuh, einen Stiefel, eine Sandale oder ggf. auch einen anderweitigen Schuhersatz, bspw. einen orthopädischen Stützschuh etc. Da der Sohlenteil mitsamt dem vorderseitig daran anschließenden und gelenkig damit verbundenen und die Großzehe abstützenden Vorderteil in etwa der Standfläche des rechten oder linken Fußes des Trägers bei flach auf dem Boden aufliegendem Fuß entsprechen kann, folgt daraus die grundsätzliche Eignung als Einlage für Schuhe oder andere Fußoberbekleidungsstücke.

Bei der Orthese ist der Gelenkabschnitt sinnvollerweise zumindest geringfügig vom Großzehengrundgelenk des auf der Orthese stehenden Fußes beabstandet. So kann sich der Gelenkabschnitt in etwa unterhalb eines Gelenks zwischen vorderen Zehenknochen und dem ersten Mittelfußknochen der Großzehe befinden, während sich das Großzehengrundgelenk beabstandet dazu im vorderen Bereich des Sohlenteils befinden kann. Eine solche Anordnung trägt der typischen Anatomie des menschlichen Fußes Rechnung und vermeidet als unangenehm empfundene Kontaktierungen von Weichteilen des Fußes mit dem Gelenkabschnitt. Insbesondere werden solchermaßen auch Druckstellen am Fuß vermieden, die durch einen zu engen Kontakt des Gelenkabschnittes mit Weichteilen des Fußes, der Großzehe und/oder sogar mit Gelenkteilen des Fußes und/oder der Großzehe führen könnten.

Bei der Orthese kann der gelenkig mit dem Sohlenteil verbundene Vorderteil zudem vorzugsweise einen Stützabschnitt zur Abstützung und Aufnahme der Unterseite der Großzehe sowie von medialen Teilbereichen der Großzehe umfassen, wobei der Stützabschnitt vorzugsweise zumindest abschnittsweise in etwa vertikal zu einem flachen unteren Abschnitt des Vorderteils ausgerichtet sein kann. So kann der Stützabschnitt des Vorderteils beispielsweise zumindest abschnittsweise einen in etwa L-förmigen Querschnitt aufweisen und solchermaßen die Großzehe zumindest in medialer Richtung abstützen, woraus sich die angestrebte Funktion der Hallux-Valgus-Korrektur ergibt.

Dass der Stützabschnitt gemeinsam mit dem Vorderteil keinen exakt L-förmigen Querschnitt aufweisen muss, wird sich weiter unten in nachfolgenden Beschreibungsteilen erschließen, denn es kann sinnvoll sein, den an der abzustützenden Großzehe anliegenden und abschnittsweise in vertikale Richtung vom Bodenteil des Vorderteils aufragenden Stützabschnitt zumindest abschnittsweise leicht konkav gewölbt zu gestalten, um eine bessere und großflächigere Kontaktierung zur Großzehe zu ermöglichen und um auch durch eine solche Maßnahme den Tragekomfort für den Benutzer der Orthese zu verbessern und die Gefahr von Druckstellen zu reduzieren.

Der Gelenkabschnitt zwischen dem Sohlenteil und dem Vorderteil ermöglicht eine mehr oder weniger normale Abrollbewegung beim Laufen und damit einen weitgehend ungestörten Bewegungsablauf bei gleichzeitig damit erzielbarer effektiver Korrektur einer Fehlstellung einer Großzehe, insbesondere einer Hallux Valgus-Fehlstellung. Wie schon erläutert, hat die erfindungsgemäße Orthese zudem den besonderen Vorteil, dass sie sich wie eine herkömmliche Schuheinlage in nahezu beliebig gestaltete Fußoberbekleidungen, Schuhe, Sandalen, Stiefel etc. einfügen lässt und dabei die gewünschte Korrekturwirkung für die Großzehe des Trägers der Orthese entfalten kann.

Die Schenkel des zumindest abschnittsweise L-förmig konturierten vorderen Stützabschnittes der Orthese können beispielsweise über einen Verbindungsabschnitt mit einem Rundungsradius verbunden sein, der in etwa einer Kontur der Großzehe folgen kann und diese dort einbettet, so dass die Orthese ohne spürbare Einschränkungen oder Komforteinbußen getragen werden kann.

Die erfindungsgemäße Orthese erlaubt es somit, mittels einer Einbettung der Großzehe im gelenkig am Sohlenteil angeordneten Vorderteil und durch ihre dort erfolgende Abstützung eine effektive Hebelwirkung auf das Großzehengrundgelenk auszuüben, was die mittels der Orthese angestrebte Korrektur der Hallux-Valgus-Fehlstellung bewirken kann.

Wahlweise kann bei einer weiteren Ausführungsvariante der Orthese eine in etwa vertikal orientierte Stützwand des Stützabschnittes elastisch verformbar ausgebildet sein, wobei insbesondere eine segmentartige Unterteilung der Stützwand eine Anpassbarkeit an eine Anatomie der Großzehe und/oder des Fußes des die Orthese verwendenden Benutzers oder Trägers ermöglicht. Die Stützwand kann bspw. durch mehrere strahlenförmig verlaufende Schlitze segmentiert und in unabhängig voneinander elastisch verformbare Zungen unterteilt sein.

Durch eine solche Segmentierung der vertikalen Stützwand, bei der strahlenförmig oder radial nach außen in Richtung zur äußeren umfangsseitigen Kante verlaufende Schlitze vorgesehen sein können, welche sich in Richtung Zentrum der Stützwand nicht treffen, sondern nur beispielsweise bis in eine Tiefe der halbrundförmig konturierten vertikalen Stützwand reichen, die einem Maß von etwa der Hälfte bis zu zwei Dritteln (oder etwas mehr) ihres Radius entsprechen kann, kann eine erhöhte Flexibilität und Anpassbarkeit des Vorderteils erreicht werden. So kann die Stützwand durch mehrere Schlitze segmentiert sein, die allesamt in etwa dieselbe Länge aufweisen können, wodurch insgesamt vier, fünf, sechs oder mehr ähnlich konturierte und im Groben ähnlich dimensionierte Zungen gebildet sein können, welche durch die Schlitze voneinander separiert sind und dadurch unabhängig voneinander elastisch ausweichen und zurückfedern können.

Durch Auswahl einer sinnvollen Materialstärke und eines geeigneten Werkstoffes für den Vorderteil weisen die Zungen, die in ihrem Verbindungsbereich schmaler sind als an ihren jeweiligen äußeren umfangsseitigen Kanten, eine definierte Elastizität und Nachgiebigkeit auf, so dass sie sich leichter an eine dort flächig anliegenden Großzehe anpassen können. Ein Teil der gesamten flächigen Kontur der Stützwand ist dadurch flexibel verformbar, wodurch der Tragekomfort für den Benutzer erheblich verbessert ist.

Wenn die Zungen oder zumindest einige der Zungen zudem die oben schon erwähnten leicht zur Großzehe hin konkav gewölbten Konturen aufweisen, ergibt sich der Vorteil einer flächigen Anlage an die Großzehe, kombiniert mit einer gewissen elastischen Verformbarkeit, was dem Stützabschnitt eine gewisse Flexibilität verleiht, so dass er sich noch besser an die Konturen und die Anatomie eines Fußes eines jeweils die Orthese tragenden Benutzers anpassen kann. Ggf. drohende Druckstellen, insbesondere verursacht durch längeres Tragen der Orthese, können somit vermieden werden.

Bei der erfindungsgemäßen Orthese kann der Gelenkabschnitt insbesondere durch ein Scharniergelenk gebildet sein, welches sich unterhalb des Zehengrundgelenks der Großzehe, aber etwas weiter in Richtung zur Großzehe befindet, wenn der Fuß auf dem Sohlenteil steht. Während das Zehengrundgelenk der Großzehe sich oberhalb des vorderen Bereiches des Sohlenteils befindet, liegt der Gelenkabschnitt weiter in Richtung zur Großzehe, so dass der verjüngte Bereich im Übergang zwischen Fußballen und Großzehe oberhalb des Gelenkabschnittes liegt. Auf diese Weise sind keine Kollisionen mit Fußsohlen oder Zehenbereichen und dem Scharnier zu erwarten.

Eine Schwenk- oder Gelenkachse des Scharniergelenks der erfindungsgemäß ausgestalteten Fuß-Orthese kann zudem gegenüber einer in etwa horizontalen Auflageebene, die ungefähr einer Trennebene zwischen einer Oberseite der Sohlenteils und einer plantaren Unterseite des Fußes entspricht, leicht angewinkelt sein. Die Schwenk- oder Gelenkachse des Scharniergelenks kann beispielsweise in einem Winkel zwischen etwa 1,5° und etwa 8° in mediale Richtung des betreffenden Fußes, der auf der Orthese steht, nach unten gegenüber der in etwa horizontalen Auflageebene, die ungefähr einer Trennebene zwischen einer Oberseite der Sohlenteils und einer plantaren Unterseite des Fußes entspricht, abgewinkelt sein, so dass die Schwenk- oder Gelenkachse zumindest leicht in laterale Richtung des Fußes ansteigt.

Insbesondere kann bei einer Ausführungsvariante der Orthese vorgesehen sein, dass die Schwenk- oder Gelenkachse des Scharniergelenks in einem Winkel zwischen etwa 3,5° und etwa 6,5° in mediale Richtung des betreffenden Fußes, der auf der Orthese steht, nach unten gegenüber der in etwa horizontalen Auflageebene, die ungefähr einer Trennebene zwischen einer Oberseite der Sohlenteils und einer plantaren Unterseite des Fußes entspricht, abgewinkelt ist, wobei der Winkel vorzugsweise in etwa 5° betragen kann.

Außerdem kann die die Schwenk- oder Gelenkachse des Scharniergelenks vorzugsweise in etwa rechtwinkelig zu einer Längsmittelachse des Sohlenteils ausgerichtet sein.

Bei einer weiteren bevorzugten Ausführungsvariante der erfindungsgemäßen Orthese kann zudem eine Längsmittelachse des Vorderteils gegenüber der Längsmittelachse des Sohlenteils leicht angewinkelt sein, und zwar vorzugsweise in spitzem Winkel. Hierdurch kann der gewünschte Korrekturwinkel der Orthese so gewählt werden, dass sich für den Träger eine optimale Korrekturwirkung ergibt.

Wenn bei der Orthese außerdem der Vorderteil mit dem Stützabschnitt vom Sohlenteil trennbar und dort anfügbar ist, so kann dies dafür genutzt werden, unterschiedliche Korrekturwinkel durch Auswahl unterschiedlicher Raststellungen einzustellen. Wenn somit bei der Orthese der Vorderteil mit dem Stützabschnitt mittels einer Rastverbindung mit dem Sohlenteil verbindbar ist, so können vorzugsweise mehrere unterschiedliche Raststellungen zur Auswahl zwischen unterschiedlichen Winkelausrichtungen ausgewählt werden.

Als Alternative zu dem oben genannten Scharniergelenk kann bei einer Variante der erfindungsgemäßen Orthese der Gelenkabschnitt wahlweise auch durch ein Filmscharnier o. dgl. Scharnier gebildet sein, welches sich sinnvollerweise ebenfalls unterhalb des Zehengrundgelenks der Großzehe, aber etwas weiter in Richtung zur Großzehe befindet, wenn der Fuß auf dem Sohlenteil steht. Während das Zehengrundgelenk der Großzehe sich oberhalb des vorderen Bereiches des Sohlenteils befindet, liegt der Gelenkabschnitt weiter in Richtung zur Großzehe, so dass der verjüngte Bereich im Übergang zwischen Fußballen und Großzehe oberhalb des Gelenkabschnittes liegt. Auf diese Weise sind keine Kollisionen mit Fußsohlen oder Zehenbereichen und dem Scharnier zu erwarten.

Da ein solches Filmscharnier allerdings sehr flach baut und im Gegensatz zu einem Scharniergelenk keine Verdickung aufweist, sondern im Gegenteil im gelenkigen Bereich normalerweise sogar dünner ausgeführt ist als in den angrenzenden Sohlen- und Auflagebereichen für den Großzeh, kann die Positionierung etwas freier gewählt werden als bei dem Scharniergelenk.

Eine Schwenk- oder Gelenkachse des Filmscharniers der erfindungsgemäß ausgestalteten Fuß-Orthese kann gegenüber einer in etwa horizontalen Auflageebene, die ungefähr einer Trennebene zwischen einer Oberseite der Sohlenteils und einer plantaren Unterseite des Fußes entspricht, leicht angewinkelt sein. Die Schwenk- oder Gelenkachse des Filmscharniers kann beispielsweise in einem Winkel zwischen etwa 1,5° und etwa 8° in mediale Richtung des betreffenden Fußes, der auf der Orthese steht, nach unten gegenüber der in etwa horizontalen Auflageebene, die ungefähr einer Trennebene zwischen einer Oberseite der Sohlenteils und einer plantaren Unterseite des Fußes entspricht, abgewinkelt sein, so dass die Schwenk- oder Gelenkachse leicht in laterale Richtung des Fußes ansteigt.

Insbesondere kann bei einer Ausführungsvariante der Orthese vorgesehen sein, dass die Schwenk- oder Gelenkachse des Filmscharniers in einem Winkel zwischen etwa 3,5° und etwa 6,5° in mediale Richtung des betreffenden Fußes, der auf der Orthese steht, nach unten gegenüber der in etwa horizontalen Auflageebene, die ungefähr einer Trennebene zwischen einer Oberseite der Sohlenteils und einer plantaren Unterseite des Fußes entspricht, abgewinkelt ist, wobei der Winkel vorzugsweise in etwa 5° betragen kann.

Außerdem kann die die Schwenk- oder Gelenkachse des Filmscharniers vorzugsweise in etwa rechtwinkelig zu einer Längsmittelachse des Sohlenteils ausgerichtet sein.

Auch bei der Ausführungsvariante der erfindungsgemäßen Orthese mit dem Filmscharnier kann eine Längsmittelachse des Vorderteils gegenüber der Längsmittelachse des Sohlenteils wahlweise leicht angewinkelt sein, und zwar vorzugsweise in spitzem Winkel. Hierdurch kann der gewünschte Korrekturwinkel der Orthese so gewählt werden, dass sich für den Träger eine optimale Korrekturwirkung ergibt.

Sofern aus Sicht des angesprochenen Fachmannes sinnvoll miteinander kombinierbar, können einige der oder alle dieser zuvor genannten Variationen oder Ausführungsvarianten der erfindungsgemäßen Orthese wahlweise auch miteinander kombiniert werden, um das oben formulierte Ziel zumindest teilweise zu erreichen, und/oder um den gewünschten Effekt der Erfindung zu erzielen.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1A zeigt eine schematische Draufsicht von oben auf eine Ausführungsvariante einer erfindungsgemäßen Orthese, die als Sohlenbauteil mit gelenkigem Vorderteil zur Aufnahme einer Großzehe eines linken menschlichen Fußes ausgestaltet ist.
Fig. 1B zeigt eine schematische Draufsicht von oben auf eine Ausführungsvariante der erfindungsgemäßen Orthese, die zur Aufnahme einer Großzehe eines rechten menschlichen Fußes ausgestaltet ist.
Fig. 1C zeigt eine schematische und perspektivische Frontansicht auf die Orthese gemäß Fig. 1A.
Fig. 1D zeigt eine schematische und perspektivische Ansicht auf eine Oberseite der Orthese gemäß Fig. 1A, bei welcher der gelenkige Vorderteil gegenüber dem Sohlenabschnitt leicht angewinkelt ist.
Fig. 2A zeigt eine schematische Draufsicht von oben auf eine weitere Ausführungsvariante der erfindungsgemäßen Orthese, die als Sohlenbauteil mit gelenkigem Vorderteil zur Aufnahme einer Großzehe eines linken menschlichen Fußes ausgestaltet ist, wobei der Vorderteil über ein Scharnier mit dem Sohlenabschnitt verbunden ist.
Fig. 2B zeigt eine schematische und perspektivische Frontansicht auf die Orthese gemäß Fig. 2A.
Fig. 2C zeigt eine schematische und perspektivische Ansicht auf eine Oberseite der Orthese, bei welcher der gelenkige Vorderteil gegenüber dem Sohlenabschnitt leicht angewinkelt ist, indem das Verbindungsscharnier zwischen den beiden Teilen leicht verschwenkt ist.
Fig. 3 zeigt eine schematische und perspektivische Explosionsdarstellung, bei der das Verbindungsscharnier, das den Vorderteil und den Sohlenabschnitt verbindet, in seine Einzelteile zerlegt ist.
Figuren 4A, 4B und 4C zeigen jeweils in schematischen Draufsichten einige unterschiedliche Möglichkeiten zur Auswahl verschiedener Winkeleinstellungen des gelenkig mit dem Sohlenabschnitt verbundenen Vorderteils, für dessen Winkelausrichtung gegenüber einer Längsmittelachse der Orthese es verschiedene Auswahlmöglichkeiten gibt.
Figuren 5A, 5B und 5C zeigen jeweils verschiedene schematische und perspektivische Detailansichten einer weiteren Ausführungsvariante der erfindungsgemäßen Orthese, bei welcher Abschnitte des Vorderteiles durch entsprechende Gestaltung flexibel an eine Großzehe eines Benutzers anpassbar sind.
Fig. 6 verdeutlicht die anatomischen Zusammenhänge und die Positionierung der Fußknochen eines auf einer rechten Fuß-Orthese stehenden rechten Fußes in Bezug auf die verschiedenen Bereiche der Orthese.

Für gleiche oder gleich wirkende Elemente der Erfindung verwendet die nachfolgende Figurenbeschreibung in der Regel jeweils gleiche Bezugsziffern. Ferner werden der Übersicht halber in vielen Fällen nur solche Bezugsziffern in den einzelnen Figuren verwendet, die für die Beschreibung der jeweiligen Figur erforderlich sind. Die dargestellten Ausführungsformen stellen lediglich Beispiele dar, wie die erfindungsgemäße Fuß-Orthese ausgestaltet sein kann und stellen keine abschließende Begrenzung dar. Auch sind die nachfolgend beschriebenen Merkmale jeweils nicht in engem Zusammenhang mit weiteren Merkmalen des jeweiligen Ausführungsbeispiels zu verstehen, sondern können jeweils im allgemeinen Zusammenhang vorgesehen sein bzw. hierfür Verwendung finden.

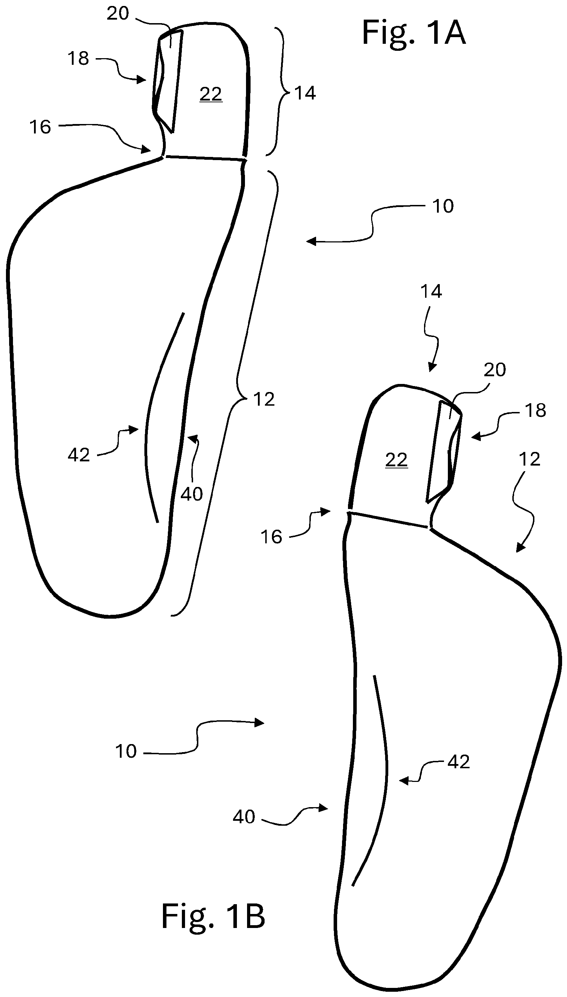

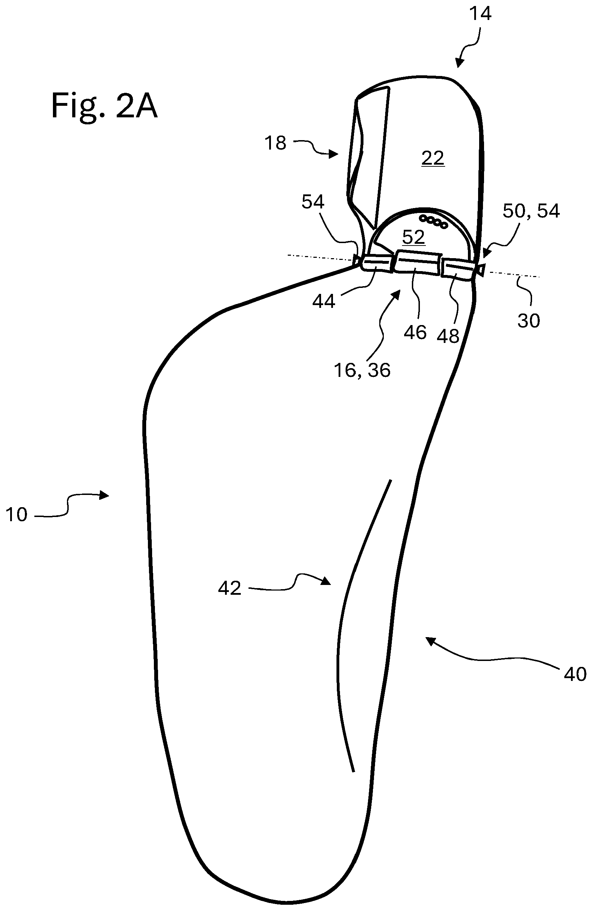

An dieser Stelle sollen zunächst anhand der ersten Ausführungsvariante der erfindungsgemäßen Fuß-Orthese gemäß den Figuren 1A bis 1C sowie der sich davon nur in einigen Details unterscheidenden zweiten Ausführungsvariante der Orthese gemäß den Figuren 2A bis 4C die wichtigsten Funktionselemente und Komponenten skizziert werden, bevor in der nachfolgenden Beschreibung alle weiteren Details und ihre jeweilige Wirkungsweise anhand der einzelnen Figuren in ausführlicherer Form erläutert werden.

So zeigt die Fig. 1A zunächst eine schematische Draufsicht von oben auf eine Ausführungsvariante einer erfindungsgemäßen Fuß-Orthese 10, die als Sohlenteil 12 mit daran gelenkig verbundenem Vorderteil 14 zur Aufnahme einer Großzehe eines menschlichen Fußes (hier nicht gezeigt) ausgestaltet ist. Die Fuß-Orthese 10, die in der weiteren Beschreibung auch verkürzt als Orthese 10 bezeichnet werden soll, weist einen dünnen Sohlenteil 12 einer Größe und einer Formgestaltung auf, die in etwa dem Umriss eines linken Fußes (ohne seine Zehen) entspricht, der oberseitig auf dem Sohlenteil 12 steht.

Eine symmetrisch dazu gestaltete Fuß-Orthese 10 für den rechten Fuß findet sich in Fig. 1B in schematischer Draufsicht wiedergegeben, was aber lediglich den Grundsatz verdeutlichen soll, dass alle nachfolgenden Aussagen, die sich auf die für den linken Fuß gestalteten Orthesen 10 beziehen, grundsätzlich auch und in gleicher Weise auf eine spiegelbildlich dazu gestaltete Orthese 10 für den rechten Fuß beziehen können.

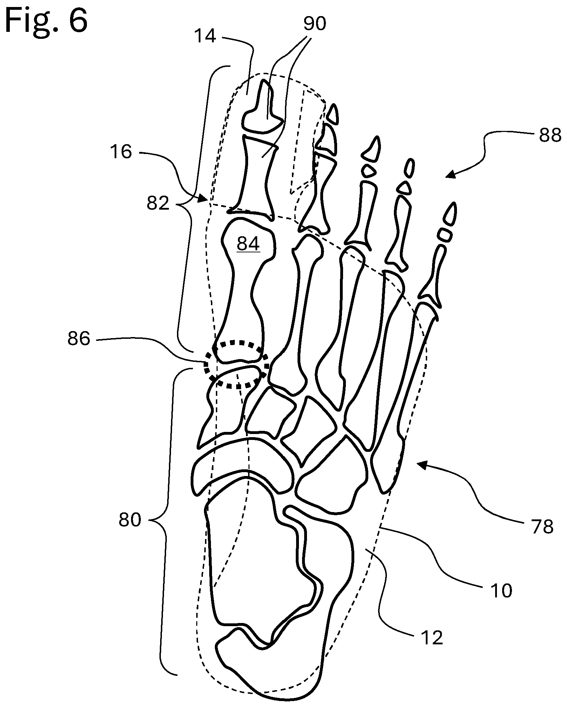

Ergänzend sei an dieser Stelle darauf hingewiesen, dass die Fig. 6 die anatomischen Zusammenhänge und die Positionierung der Fußknochen eines auf einer rechten Fuß-Orthese 10 stehenden rechten Fußes in Bezug auf die verschiedenen Bereiche der Orthese 10 verdeutlicht. Dort sind zudem die Anordnung des Gelenkabschnittes 16 in Bezug auf das Großzehengrundgelenk und die bevorzugten Abstände zwischen den beiden Gelenken (d.h. des Fußes und der Orthese) verdeutlicht.

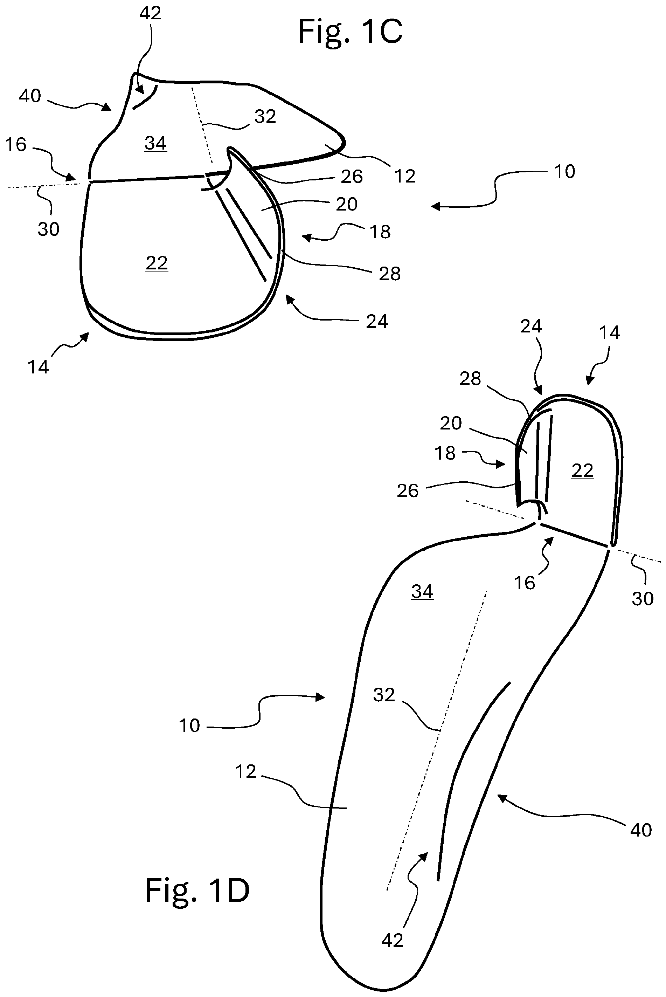

Der mittels eines beweglichen Gelenkabschnittes 16 an einer vorderen Stirnseite des Sohlenteils 12 angeordnete Vorderteil 14 weist in etwa eine Größe und eine Formgebung auf, dass darauf die Großzehe des auf dem Sohlenteil 12 befindlichen Fußes (nicht gezeigt) aufliegen kann. Der Gelenkabschnitt 16 befindet sich dabei nicht genau unterhalb eines Zehengrundgelenks (hier ebenfalls nicht gezeigt, vgl. aber Fig. 6), sondern etwas weiter in Richtung zur Fußspitze vor dem Zehengrundgelenk, so dass ein Abknicken der Großzehe im Gelenk zwischen erstem Mittelfußknochen und dem Zehenknochen der Großzehe mit einer entsprechenden Schwenkbewegung im darunterliegenden Gelenkabschnitt 16 einhergeht, was anhand der Fig. 1D veranschaulicht ist. Wichtig ist dabei, dass der Gelenkabschnitt 16 an einer Stelle positioniert ist, wo es zu keinen Druckstellen an der Fußunterseite kommen kann, nämlich vorzugsweise im Übergangsbereich zwischen Fußballen und Großzehe.

So zeigt die Fig. 1D eine schematische und perspektivische Ansicht auf die Oberseite der Orthese 10. Dort ist der Vorderteil 14 im Gelenkabschnitt 16 um einen Winkel von etwa 20 bis 30 Grad gegenüber dem Sohlenteil 12 abgewinkelt, was einer natürlichen Abrollbewegung des menschlichen Fußes beim Gehen entspricht, wo bei jedem einzelnen Schritt das Abheben der Ferse und der Sohle vom Boden mit einem Abwinkeln der Großzehe und ihrem Anheben nach oben einhergeht.

Unabhängig von der Ausgestaltung des Gelenkabschnittes 16 ist jederzeit ausreichend Platz für Scharniergelenke oder dergleichen, wie sie in den Figuren 2A ff. beispielhaft gezeigt sind, da die Großzehe nur mit ihrer voluminöseren Unterseite auf dem Vorderteil 14 aufliegt, während im Bereich vorderhalb des Zehengrundgelenks ausreichend Raum für die Gestaltung des Gelenkabschnittes 16 verbleibt. Die Fußsohle liegt normalerweise auch im Bereich der vorderen Fußballen (nicht gezeigt) auf dem Sohlenteil 12 auf, nicht jedoch im Bereich vorderhalb der Zehengrundgelenke, so auch nicht mit dem Zehengrundgelenk der Großzehe.

Hinsichtlich der genaueren anatomischen Zusammenhänge bei einem menschlichen Fuß sei wiederum auf die Fig. 6 verwiesen.

Wie es die Fig. 1C deutlich erkennen lässt, ist der über den Gelenkabschnitt 16 stirnseitig am Sohlenteil 12 gelenkig verankerte Vorderteil 14 im Querschnitt L-förmig gestaltet, da ein vertikaler Stützabschnitt 18 in Gestalt einer in etwa vertikal geformten Stützwand 20 gegenüber dem flachen unteren Abschnitt 22 des Vorderteils 14, der am Gelenkabschnitt 16 ansetzt, nach oben aufragt und die Großzehe einseitig anliegen lässt und dadurch abstützt. In der dargestellten linken Orthese 10 für den linken Fuß, wie sie jeweils in den Figuren 1A, 1C und 1D gezeigt ist, befindet sich der durch die Stützwand 20 gebildete Stützabschnitt 18 am linken Rand des Vorderteils 14 und stützt somit die mediale Seite der linken Großzehe, d.h. die beim zu behandelnden Beschwerdebild des Hallux Valgus zur linken Körperaußenseite strebende Seite der Großzehe, an die sich die weiteren Zehen des linken Fußes anschließen.

In der in Fig. 1B gezeigten rechten Orthese 10 für den rechten Fuß befindet sich dagegen die Stützwand 18 am rechten Rand des Vorderteils 14 und stützt somit wiederum die mediale Seite der rechten Großzehe, d.h. die beim zu behandelnden Beschwerdebild des Hallux Valgus zur rechten Körperaußenseite strebende Seite der Großzehe, an die sich die weiteren Zehen des rechten Fußes anschließen.

Wie es die schematische und perspektivische Frontansicht auf die Orthese 10 der Fig. 1C erkennen lässt, kann die Stützwand 20 vorzugsweise in einem sanften Rundungsradius 24 vom flachen unteren Abschnitt 22 des Vorderteils 14 übergehen und nach oben hin ebenfalls in sanfter Rundung 26 auslaufen. Diese obere Rundung 26 der Stützwand 20 zieht diese vorzugsweise leicht nach innen zur Großzehe hin, so dass deren Konturen an ihrer medialen Seite besser gefolgt ist und die Großzehe vom Stützabschnitt 18 teilweise umfangen und abgestützt ist. Damit wölbt sich die Stützwand 20 zur Großzehe hin in konkaver oder zumindest abschnittsweise konkaver Form.

Eine vordere stirnseitige Kante 28 des Stützabschnittes 18 bzw. der Stützwand 20 kann in der gezeigten Weise angeschrägt sein, so dass die Stützwand 20 in ihrem mittleren Bereich nicht ganz die Länge des flachen unteren Abschnittes 22 des Vorderteils 14 aufweist.

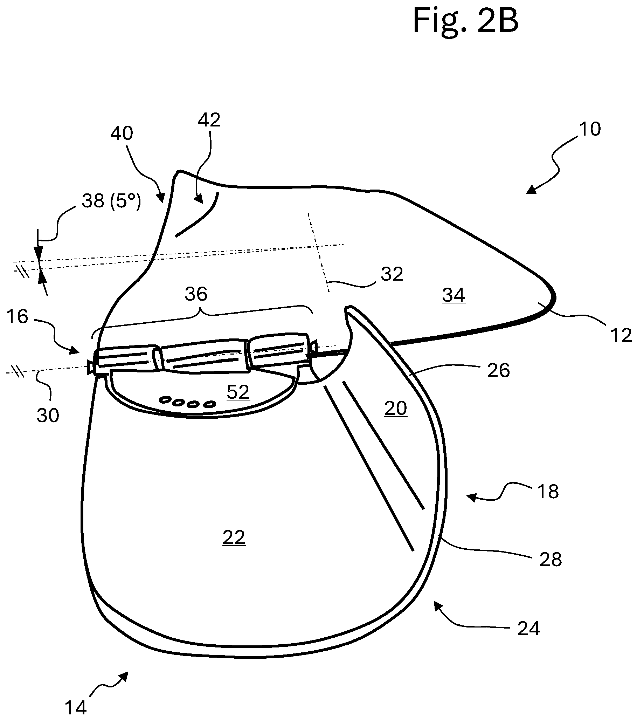

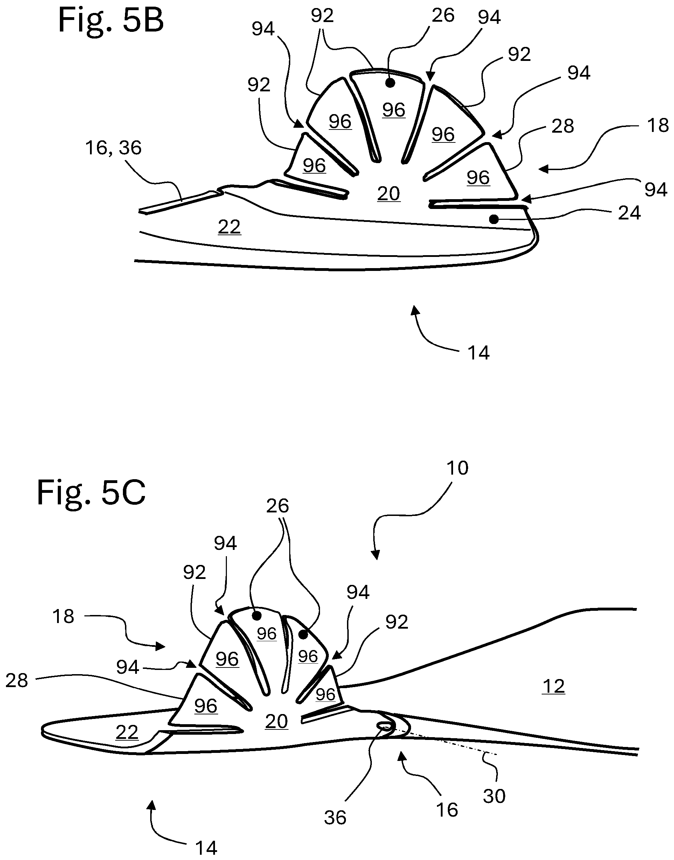

Es sei an dieser Stelle betont, dass über die konkrete Ausgestaltung des Gelenkabschnittes 16, der eine Schwenkbewegung des Vorderteils 14 gegenüber dem Sohlenteil 12 in der in Fig. 1D angedeuteten Weise ermöglicht, an dieser Stelle, d.h. unter Bezugnahme auf die Figuren 1A bis 1D, noch keine Aussage getroffen sein soll. Wichtig zu betonen ist zunächst nur die Beweglichkeit des Vorderteils 14 im Gelenkabschnitt 16 mit einer Schwenkachse 30, die in etwa quer zu einer Längsmittelachse 32 der Fuß-Orthese 10 und in etwa parallel zur flachen Oberfläche 34 des Sohlenteils 12 liegt. Dass diese Schwenkachse 30, die in den Figuren 1C und 1D jeweils durch strichpunktierte Linierung angedeutet ist, leicht zur Horizontalen abgewinkelt sein sollte, wird im Detail erst weiter unten beschrieben und lässt sich besser anhand der Fig. 2B oder auch anhand der Fig. 5C veranschaulichen.

Die Art des Gelenks, das den Gelenkabschnitt 16 bildet, kann unterschiedlich ausgeführt sein, so etwa als Scharniergelenk, wie es anhand der nachfolgenden Figuren 2A ff. beispielhaft gezeigt ist. Das Gelenk kann jedoch auch durch eine entsprechende Gestaltung der Orthese 10 und ihres Aufbaus realisiert sein, beispielsweise durch einen nachgiebigen Abschnitt im Verbindungsbereich zwischen dem steiferen Sohlenteil 12 und dem ebenfalls steifer ausgeführten Vorderteil 14, so dass sich die gewünschte Beweglichkeit im Gelenkabschnitt 16 durch eine geeignete Materialauswahl und durch entsprechende Fertigungsmethoden ergeben kann. Eine solche Gestaltung des Gelenkabschnittes 16 lässt sich beispielsweise durch ein Mehrkomponenten-Spritzgießverfahren realisieren, wahlweise auch durch eine Reduzierung der Schichtdicke der Bauteile an der betreffenden Stelle, unterstützt ggf. durch eine flexible Materialeinlage, die für die gewünschte Belastbarkeit bei gleichzeitiger Beweglichkeit sorgen kann.

Ein solchermaßen ausgestalteter Gelenkabschnitt 16 kann beispielsweise durch ein Filmscharnier gebildet werden. So können die Figuren 1A bis 1D wahlweise so verstanden werden, dass der dort schematisch angedeutete Gelenkabschnitt 16 zwischen dem Sohlenteil 12 und dem Vorderteil 14 als durchgängige Materialbrücke ausgebildet ist, gebildet beispielsweise durch ein solches Filmscharnier. Wahlweise können die Figuren 1A bis 1D jedoch auch in der Weise verstanden werden, wie dies durch die folgenden Figuren 2A ff. in konkreter Ausgestaltung verdeutlicht wird, nämlich in einer Ausgestaltung des Gelenkabschnittes 16 mit einem Scharniergelenk oder Verbindungsscharnier 36.

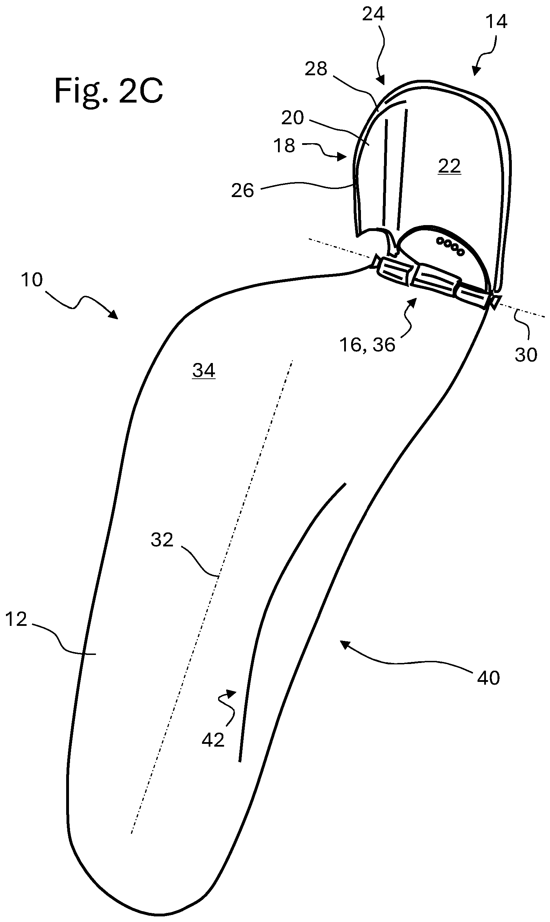

So ist eine weitere Ausführungsvariante der erfindungsgemäßen Fuß-Orthese 10 in den Figuren 2A, 2B und 2C in verschiedenen Ansichten gezeigt. Dort zeigt die Fig. 2A eine schematische Draufsicht von oben auf diese weitere Ausführungsvariante der erfindungsgemäßen Orthese 10, die als Sohlenteil 12 mit gelenkigem Vorderteil 14 zur Aufnahme der linken Großzehe eines linken menschlichen Fußes (hier nicht gezeigt) ausgestaltet ist, wobei der Vorderteil 14 über ein Verbindungsscharnier 36 mit dem flachen Sohlenabschnitt oder Sohlenteil 12 verbunden ist.

Die Fig. 2B zeigt eine schematische und perspektivische Frontansicht auf die Orthese 10 gemäß Fig. 2A. Weiterhin zeigt die Fig. 2C eine schematische und perspektivische Ansicht auf eine Oberseite der Orthese 10, bei welcher der gelenkige Vorderteil 14 gegenüber dem Sohlenabschnitt 12 leicht angewinkelt ist, indem das Verbindungsscharnier 36 zwischen den beiden Teilen 12 und 14 leicht verschwenkt ist.

Die in den Figuren 2A bis 2C gezeigte Ausführungsvariante der Orthese 10 unterscheidet sich im Grundaufbau nicht von der ersten Variante, wie sie oben unter Bezugnahme auf die Figuren 1A bis 1D beschrieben wurde, hat gegenüber dieser allerdings ein Verbindungsscharnier 36 im Gelenkabschnitt 16, während dieser Gelenkabschnitt 16 bei der ersten Variante als undefiniert und variabel gestaltbar beschrieben wurde. Aus diesem Grund sollen in den nachfolgenden Beschreibungspassagen im Wesentlichen der Aufbau und die Funktionsweise dieses als Gelenkabschnitt 16 dienenden Verbindungsscharniers 36 erläutert werden, während zu den übrigen Bestandteilen der Orthese 10 ergänzend auf die obigen Beschreibungsstellen verwiesen werden kann.

Somit gilt im Wesentlichen alles oben zur ersten Ausführungsvariante Gesagte gleichermaßen zur zweiten Ausführungsvariante gemäß Figuren 2A bis 2C, mit Ausnahme der Detailbeschreibungen zur konkreten Ausgestaltung und Anordnung des Scharniergelenks 36, welches den Gelenkabschnitt 16 bildet. Doch selbst hinsichtlich der Wirkungsweise des Scharniergelenks 36 ergeben sich keine wesentlichen Unterschiede, so dass auch die in den Figuren 2A bis 2C verdeutlichte Schwenkachse 30 grundsätzlich dieselbe sein kann wie bei der ersten Ausführungsvariante gemäß Figuren 1A bis 1D.

In gleicher Weise, wie dies oben schon erläutert wurde, wäre eine symmetrisch dazu gestaltete Fuß-Orthese 10 für den rechten Fuß in entsprechender Weise geformt, wie dies mit der Fig. 1B anhand des ersten Ausführungsbeispiels gezeigt ist. Auch bei der zweiten Ausführungsvariante gemäß Figuren 2A bis 2C sollen sich alle Aussagen, die Bezug nehmen auf die für den linken Fuß gestalteten Orthesen 10, grundsätzlich auch und in gleicher Weise auf eine spiegelbildlich dazu gestaltete Orthese 10 für den rechten Fuß gelten.

Wiederum weist auch bei der zweiten Ausführungsvariante der Orthese 10 der mittels des um die Gelenkachse 30 schwenkbeweglichen Scharniergelenks 36 an der vorderen Stirnseite des Sohlenteils 12 angeordnete Vorderteil 14 in etwa die Größe und Formgebung auf, dass darauf die Großzehe des auf dem Sohlenteil 12 befindlichen Fußes (nicht gezeigt) aufliegen kann. Das schlank bauende Scharniergelenk 36 des Gelenkabschnittes 16 befindet sich dabei in etwa in einem Bereich unterhalb und vorderhalb eines Zehengrundgelenks (hier ebenfalls nicht gezeigt), so dass ein Abknicken der Großzehe im Gelenk zwischen dem Mittelfußknochen und dem Zehenknochen der Großzehe (vgl. hierzu Fig. 6) durch eine entsprechende Schwenkbewegung im darunter und davor liegenden Scharniergelenk 36 ermöglicht ist, was anhand der Fig. 2C veranschaulicht ist.

So zeigt die Fig. 2C eine schematische und perspektivische Ansicht auf die Oberseite der zweiten Ausführungsvariante der Fuß-Orthese 10 mit dem als Scharniergelenk 36 ausgebildeten Gelenkabschnitt 16. Dort ist der Vorderteil 14 im Gelenkabschnitt 16 mittels des um die Gelenkachse 30 um einen Winkel von etwa 20 bis 30 Grad gegenüber dem Sohlenteil 12 abgewinkelt, was einer natürlichen Abrollbewegung des menschlichen Fußes beim Gehen entspricht, wo bei jedem einzelnen Schritt das Abheben der Ferse und der Sohle des Fußes vom Boden mit einem Abwinkeln der Großzehe und ihrem Anheben nach oben einhergeht.

Entsprechend der gezeigten Ausgestaltung des Gelenkabschnittes 16 als schlank bauendes Scharniergelenk 36 ist aufgrund der Anatomie des menschlichen Fußes, seiner Zehen und seiner Gelenke ausreichend Platz für das Scharniergelenk 36, ohne dass es mit dem Bereich des Fußes, der zwischen Zehengrundgelenk und der eigentlichen Großzehe liegt, kollidiert oder auch nur in stärkeren Kontakt kommt, da die Großzehe nur mit ihrer voluminöseren Unterseite auf dem Vorderteil 14 aufliegt, während im Bereich vorderhalb des Zehengrundgelenks ausreichend Raum für die Gestaltung des schlanken Scharniergelenks 36 verbleibt.

Wie es wiederum die Fig. 2B deutlich erkennen lässt, ist der über das Scharniergelenk 36 des Gelenkabschnittes 16 stirnseitig am Sohlenteil 12 gelenkig verankerte Vorderteil 14 im Querschnitt L-förmig gestaltet, da der vertikale Stützabschnitt 18 in Gestalt der in etwa vertikal geformten Stützwand 20 gegenüber dem flachen unteren Abschnitt 22 des Vorderteils 14, der am Scharniergelenk 36 ansetzt, nach oben aufragt und die Großzehe einseitig anliegen lässt und dadurch abstützt. Der Vorderteil 14 der zweiten Ausführungsvariante unterscheidet sich hinsichtlich seiner Gestaltung somit nicht vom Vorderteil gemäß erster Variante, so dass hinsichtlich der Figuren 1C und 2B im Wesentlichen dasselbe gilt. In der dargestellten linken Orthese 10 für den linken Fuß, wie sie jeweils in den Figuren 2A, 2B und 2C gezeigt ist, befindet sich der durch die Stützwand 20 gebildete Stützabschnitt 18 am linken Rand des Vorderteils 14 und stützt somit die mediale Seite der linken Großzehe, d.h. die beim zu behandelnden Beschwerdebild des Hallux Valgus zur linken Körperaußenseite strebende Seite der Großzehe, an die sich die weiteren Zehen des linken Fußes anschließen.

Wie schon weiter oben erläutert, ist die Beweglichkeit des Vorderteils 14 im Gelenkabschnitt 16 durch das Scharniergelenk 36 mit seiner Schwenkachse 30 gewährleistet. Die Schwenkachse 30 liegt in etwa quer zur Längsmittelachse 32 der Fuß-Orthese 10 und in etwa parallel zur flachen Oberfläche 34 des Sohlenteils 12 (vgl. hierzu die Figuren 2B und 2C). Wie ebenfalls weiter oben schon erläutert, ist die Schwenkachse 30, die durch das Scharniergelenk 36 verläuft und dessen Beweglichkeit definiert, leicht zur Horizontalen abgewinkelt, und zwar typischerweise in einem Winkel von etwa fünf Grad.

Die Fig. 2B zeigt diesen Winkel als Neigungswinkel 38 der Gelenkachse 30 gegenüber der Oberfläche 34 des Sohlenteils 12, wobei der typische Neigungswinkel von etwa 5° ebenfalls dort eingezeichnet ist. Der besseren Übersichtlichkeit halber wurde oberhalb der durch das Scharniergelenk 36 verlaufenden Gelenkachse 30 eine dazu parallele Linie eingezeichnet, zu welcher eine parallel zur Oberfläche 34 des Sohlenteils 12 verlaufende strichpunktierte Linie in Bezug gesetzt wurde, so dass zwischen diesen beiden strichpunktierten Linien der Neigungswinkel 38 von ca. 5° eingezeichnet werden konnte.

Wie es die Fig. 2B deutlich erkennen lässt, ist der Neigungswinkel 38 in medialer Richtung des Fußes (in Fig. 2B nach links weisend) nach unten gerichtet, während er in lateraler Richtung, d.h. zur Körperaußenseite des linken Fußes gerichtet (in Fig. 2B nach rechts weisend) ansteigt. Dieser in medialer Richtung nach unten abfallende Neigungswinkel 38 unterstützt im Zusammenhang mit einer leichten Unterstützung einer inneren Längswölbung der Fußsohle die gewünschte Korrektur der Zehenfehlstellung, insbesondere im Zusammenhang mit der am Stützabschnitt 18 anliegenden Großzehe des solchermaßen abgestützten Fußes.

Es sei an dieser Stelle darauf hingewiesen, dass die angegebene Winkelstellung von etwa 5° nicht einschränkend zu verstehen ist, sondern lediglich beispielhaft. Auch andere Winkel zwischen etwa 1,5° und bis zu 8° oder wenig mehr können für den angestrebten Zweck sinnvoll sein, wobei sich allerdings Winkelbereiche um ca. 5° als besonders sinnvoll und vorteilhaft erwiesen haben.

An der medialen Längsseite 40 des Sohlenteils 12 ist eine leichte Auswölbung 42 nach oben zu erkennen, die über die Oberfläche 34 des Sohlenteils 12 hinausragt, so dass sie die gewünschte innere Längswölbung der Fußsohle unterstützt und dem Fuß die gewünschte Lage vermittelt, in welcher die angestrebte Korrektur der Zehenfehlstellung der Großzehe des auf der Orthese 10 stehenden Fußes realisierbar ist. Diese lediglich durch eine gekrümmte Linie angedeutete Auswölbung 42 nach oben, die sich an der medialen Längsseite 40 des Sohlenteils 12 befindet, ist jeweils in den Figuren 1A bis 2C zeichnerisch wiedergegeben.

Wie es die Figuren 2A bis 2C jeweils verdeutlichen, ist das Scharniergelenk 36 durch insgesamt drei in Fluchtung zueinander angeordnete Hülsenabschnitte 44, 46 und 48 gebildet, in denen sich ein eingeschobener schlanker Gelenkbolzen 50 befindet, der alle drei in Fluchtung befindlichen Hülsenabschnitte 44, 46 und 48 durchdringt und durch welchen Gelenkbolzen 50 die Gelenkachse 30 des Scharniergelenks 36 mittig verläuft. Somit ist der Gelenkbolzen 50, dessen Außendurchmesser unter geringem Spiel in etwa mit den zylindrischen Innenmantelflächen der Hülsenabschnitte 44, 46 und 48 korrespondiert, auch koaxial zu diesen Hülsenabschnitten 44, 46 und 48 angeordnet.

Alle entsprechenden Bezugsziffern der einzelnen Teile des Scharniergelenks 36 sind nur in der Fig. 2A eingezeichnet, um die beiden anderen Ansichten der Figuren 2B und 2C nicht zu überfrachten. Allerdings sind dort alle in Fig. 2A bezeichneten Teile ebenfalls erkennbar, auch wenn sie nicht mit den entsprechenden Bezugsziffern kenntlich gemacht sind. Die beiden äußeren Hülsenabschnitte 44 und 48 sind jeweils fest an der vorderen flachen Stirnseite des Sohlenteils 12 verankert, beispielsweise einteilig im Materialverbund mit dem Sohlenteil 12 ausgebildet, was etwa durch ein entsprechendes Formungsverfahren für einen spritzgegossenen Sohlenteil 12 erfolgt sein kann. Damit stellen die beiden äußeren Hülsenabschnitte 44 und 48 gemeinsam den zentralen Stütz- und Ankerpunkt für den Gelenkbolzen 50 dar, dessen dazwischen befindlicher mittlerer Abschnitt den mittleren Hülsenabschnitt 46 trägt, an dem wiederum der Vorderteil 14 gehalten ist.

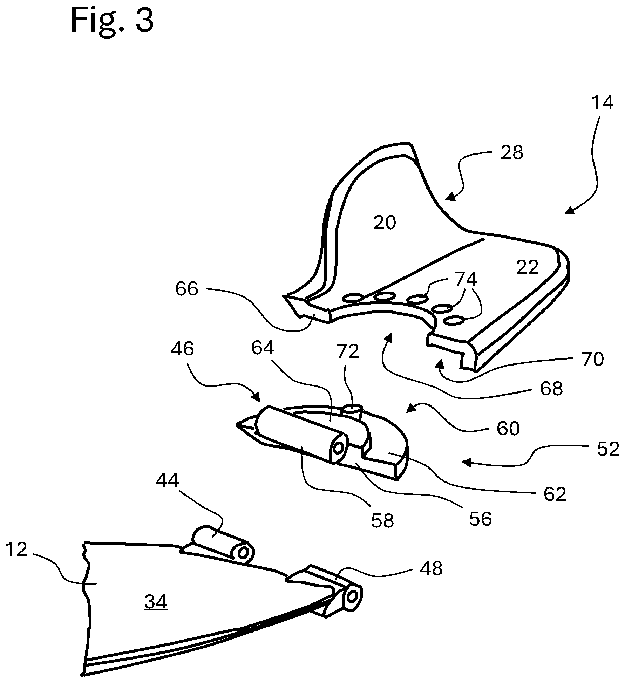

Dass der mehrteilig aufgebaute Vorderteil 14 je nach gewählter Ausführungsvariante von einem Verbindungsstück 52 abgenommen und in unterschiedlichen Winkelstellungen wieder damit verbunden werden kann, soll anhand der Detaildarstellung der Fig. 3 veranschaulicht werden, während an dieser Stelle der Hinweis genügen soll, dass eine solche optionale Trennbarkeit des Verbindungsstückes 52 vom Vorderteil 14 als bevorzugte Ausführungsvariante ermöglicht sein kann, jedoch nicht zwingend gegeben sein muss. Sofern das in Fig. 2A mit der Bezugsziffer 52 bezeichnete Verbindungsstück einen untrennbaren Bestandteil des Vorderteils 14 bildet, so ist keine solche Verstellbarkeit gegeben, sondern lediglich die beschriebene Verschwenkbarkeit des Vorderteils 14 um die Gelenkachse 30 gegenüber dem flachen Sohlenteil 12.

Endseitig können am Gelenkbolzen 50 jeweils verdickte Bolzenköpfe 54 angeordnet sein, die ein seitliches Herausgleiten des Gelenkbolzens 50 aus den Hülsenabschnitten 44, 46 und 48 verhindern können. Wahlweise kann einer dieser Bolzenköpfe 54 auch nachträglich angebracht sein, um den Gelenkbolzen 50 nach seiner Montage beim Zusammenbau des Scharniergelenks 36 an seinem vorgesehenen Platz zu halten.

Somit ist das Scharniergelenk 36 solchermaßen aufgebaut, dass sich der mittlere Hülsenabschnitt 46 mit dem darin befindlichen Gelenkbolzen 50 in Fluchtung mit den äußeren Hülsenabschnitten 44 und 48 befindet, die ebenfalls vom Gelenkbolzen 50 durchdrungen sind, so dass der mit dem Vorderteil 14 verbundene mittlere Hülsenabschnitt 46 gegenüber den beiden äußeren Hülsenabschnitten 44 und 48 verschwenkt werden kann. In der Fig. 2A bezeichnet die Bezugsziffer 44 den am Sohlenteil 12 verankerten linken äußeren Hülsenabschnitt, während die Bezugsziffer 48 den ebenfalls am Sohlenteil 12 verankerten rechten äußeren Hülsenabschnitt bezeichnet. Die beiden äußeren Hülsenabschnitte 44 und 48 können wahlweise etwas kürzer ausfallen als der vorzugsweise etwas längere mittlere Hülsenabschnitt 46, da der Gelenkbolzen 50 lagestabil in den äußeren Hülsenabschnitten 44 und 48 gehalten ist.

Der mittlere Hülsenabschnitt 46 kann beispielsweise in etwa fingerbreit ausfallen und einen äußeren Durchmesser von vorzugsweise weniger als fünf Millimeter haben. Die beiden äußeren Hülsenabschnitt 44 und 48 können beispielsweise in etwa die halbe Breite des mittleren Hülsenabschnittes 46 oder etwas mehr haben und messen in ihren äußeren Durchmessern ebenfalls vorzugsweise weniger als fünf Millimeter. Sinnvollerweise unterscheiden sich die Hülsenabschnitte 44, 46 und 48 lediglich in ihren Längen, weisen aber gleich Innendurchmesser und in etwa gleiche Außendurchmesser auf.

Die Fig. 3 zeigt eine schematische und perspektivische Explosionsdarstellung, bei der das Scharniergelenk 36, das den Vorderteil 14 und den Sohlenabschnitt 12 gelenkig verbindet, in seine Einzelteile zerlegt ist. Links unten ist ein vorderer Abschnitt des Sohlenteils 12 erkennbar, an dessen vorderer Schmalseite die beiden äußeren Hülsenabschnitt 44 und 48 angeformt sind. Zwischen den beiden miteinander fluchtenden Hülsenabschnitten 44 und 48 befindet sich eine Lücke, deren Maß das Einfügen des mittleren Hülsenabschnittes 46 ermöglicht, der aber in der Darstellung der Fig. 3 nach oben verschoben ist. Der die Hülsenabschnitte 44, 46 und 48 normalerweise verbindende Gelenkbolzen 50 ist herausgezogen und entfernt.

Am mittleren Hülsenabschnitt 46, der aus seiner Positionierung zwischen den äußeren Hülsenabschnitten 44 und 48 des Sohlenteils 12 entnommen ist, befindet sich das oben schon erwähnte scheibenförmige Verbindungsstück 52, wobei das Verbindungsstück 52 eine in etwa halbkreisförmige Kontur mit gestufter Stärke aufweist. An der geraden oder ggf. einen Öffnungswinkel von etwas weniger als 180° beschreibenden Grundseite 56 des halbkreisförmigen Verbindungsstückes 52 ist der mittlere Hülsenabschnitt 46 angeformt, und zwar in einer Verbindung über einen Abschnitt seines gesamten Außenmantels 58.

Die von der Grundseite 56 mit dem daran angeordneten mittleren Hülsenabschnitt 46 abgewandte halbkreisförmige Vorderseite 60 des Verbindungsstückes 52 umfasst eine untere Stufe 62 mit größerem Radius und eine darauf befindliche obere Stufe 64 mit kleinerem Radius. Beide Stufen 62 und 64 liegen aufeinander bzw. bilden eine Ausformung einer oberen Flachseite des Verbindungsstückes 52 während die untere Flachseite eben gestaltet sein kann und keine Verbindungsfunktion zum Vorderteil hat. Eine Gesamtstärke des flachen und in der beschriebenen Weise oberseitig gestuften Verbindungsstückes 52 kann der Materialstärke des flachen unteren Abschnittes 22 des Vorderteils 14 entsprechen.

Eine Unterseite des flachen unteren Abschnittes 22 des Vorderteils 14 weist eine mit der Kontur des Verbindungsstückes 52 korrespondierende Ausformung auf, ebenso wie die von der vorderen stirnseitigen Kante 28 der Stützwand 20 abgewandte Rückseite 66 des Vorderteils 14. In dieser Rückseite 66 befindet sich eine halbkreisförmige Aussparung 68, deren Größe und Radius mit der Größe und der Kontur der oberen Stufe 64 am halbkreisförmigen und scheibenförmigen Verbindungsstück 52 korrespondiert, so dass die Teile ineinandergefügt werden können, was die Figuren 2A bis 2C jeweils erkennen lassen.

Unterseitig am Vorderteil setzt sich eine muldenförmige Aussparung 70 fort, die in ihrer Kontur und in ihren Dimensionen mit der Kontur und Größe der unteren Stufe 62 des Verbindungsstückes 52 korrespondiert, so dass dieses formschlüssig und mit geringem Spiel dort angefügt und die Teile ineinandergefügt werden können, wie dies die Figuren 2A bis 2C jeweils erkennen lassen.

Schließlich ragt in etwa mittig an der unteren Stufe 62 des Verbindungsstückes 52 ein zylindrischer Zapfen 72 nach oben, der eine Länge aufweist, so dass er die Oberseite der oberen Stufe 64 nicht überragt. Der Zapfen 72 ist so dimensioniert, dass er in entsprechend dimensionierte hohlzylindrische Durchbrüche 74 im Vorderteil 14 eingreifen kann, wobei dort vorzugsweise eine Verrastung stattfindet, wenn der Zapfen 72 in eine der im Vorderteil 14 vorgesehenen Durchbrüche 74 eingreift. Wie es die Fig. 3 erkennen lässt, kann der Vorderteil beispielsweise mit fünf solchen Durchbrüchen 74 ausgestattet sein, die gleichmäßig über den Verlauf der halbkreisförmigen Aussparung 68 verteilt und von deren Rand ebenso weit beabstandet sind, wie der Abstand des Zapfens 72 vom Rand der oberen Stufe 64 beträgt.

Auf diese Weise stellen die insgesamt fünf Durchbrüche 74 fünf verschiedene Rastpositionen für den Zapfen 72 zur Verfügung, wobei die Durchbrüche 74 beispielsweise in der gezeigten Weise einen maximalen Öffnungswinkel der außen liegenden Durchbrüche 74 von etwas mehr als 90° definieren können. Die Figuren 2A bis 2C zeigen dagegen nur insgesamt vier eng beieinanderliegende Durchbrüche 74, die einen kleineren Öffnungswinkel von deutlich weniger als 90° definieren.

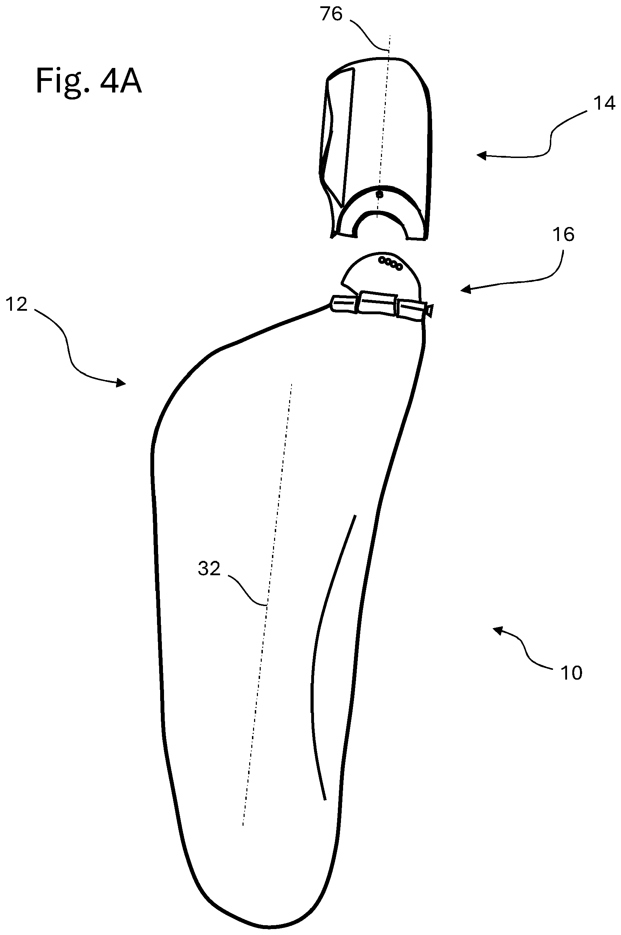

Entsprechend den Darstellungen der Figuren 2A bis 2C und den drei schematischen Darstellungen der Figuren 4A bis 4C kann die Gestaltung der Durchbrüche 74 und des Zapfens 72 sowie der oberen und unteren Stufen 62 und 64 wahlweise auch in kinematischer Umkehr erfolgen, da dort am Verbindungsstück 52 die Durchbrüche 74 erkennbar sind, während sich der Zapfen 72 am Vorderteil 14 befindet. Die Wirkungsweise bleibt dabei jedoch unverändert, weil die nebeneinander befindlichen Durchbrüche 74 je nach Positionierung und Verrastung des Zapfens 72 unterschiedliche Winkelstellungen des Vorderteils 14 in Bezug auf die Längserstreckungsrichtung bzw. die Ausrichtung der Längsmittelachse 32 des Sohlenteils 12 erlauben.

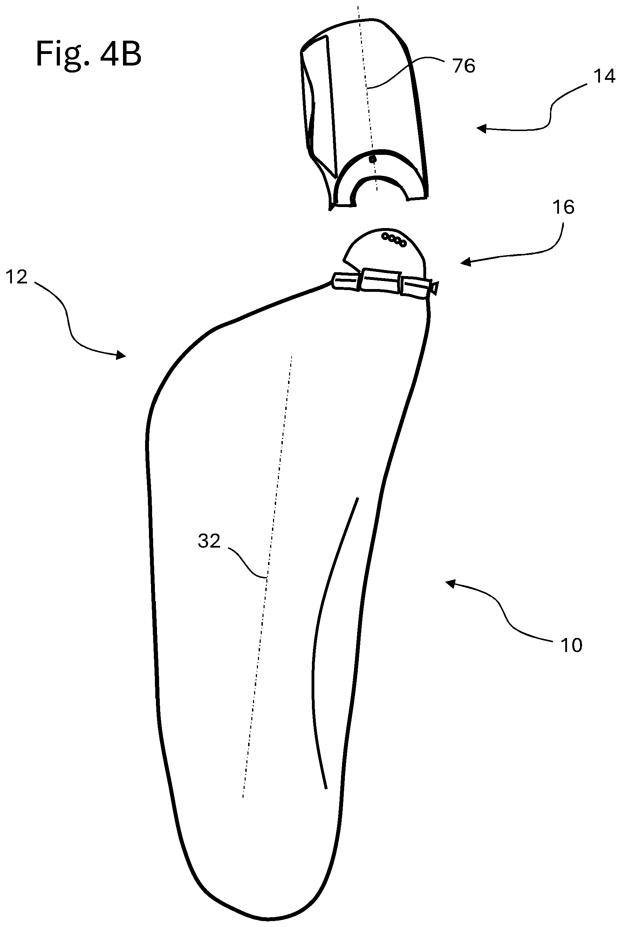

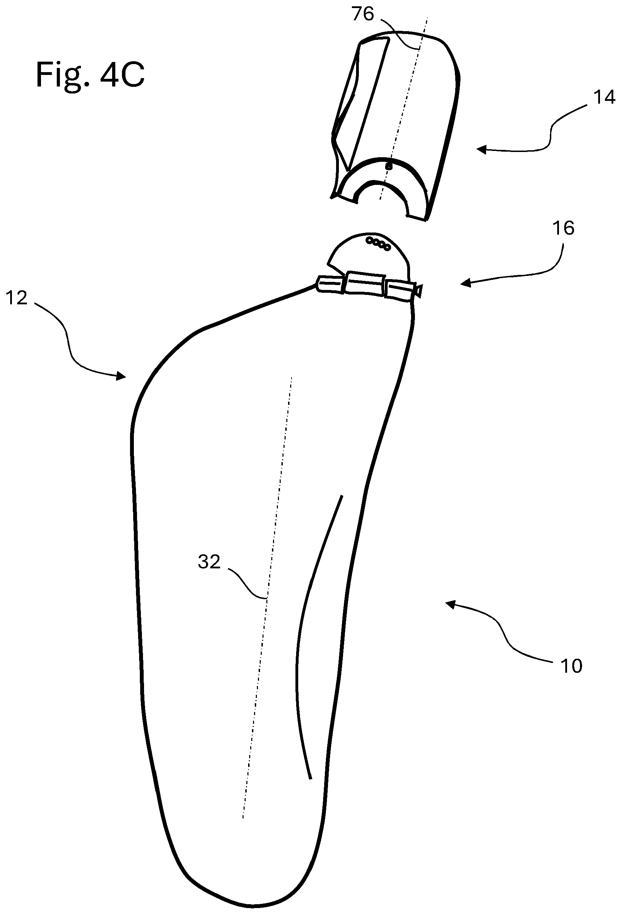

Die Figuren 4A, 4B und 4C zeigen jeweils in schematischen Draufsichten einige unterschiedliche Möglichkeiten zur Auswahl verschiedener Winkeleinstellungen des gelenkig mit dem Sohlenabschnitt 12 verbundenen Vorderteils 14, für dessen Winkelausrichtung gegenüber der Längsmittelachse 32 der Orthese 10 es auf Grundlage der verschiedenen Positionen der nebeneinander angeordneten Durchbrüche 74 und des entsprechend positionierbaren Zapfens 72 verschiedene Auswahlmöglichkeiten gibt. Die Vorderteile 14 sind jeweils von den Verbindungsstücken 52 getrennt und können wahlweise in einer der gezeigten Winkelstellungen dort angefügt werden.

Die verschiedenen Ausrichtungen der Längserstreckungsrichtung des Vorderteils 14 lassen sich an den mit entsprechenden Linierungen verdeutlichten Längsmittelachsen 76 der in unterschiedliche Rastpositionen gebrachten Vorderteile 14 ablesen, die jeweils in Bezug auf die Längsmittelachse 32 des Sohlenteils 12 zu betrachten sind.

So zeigt beispielsweise die Fig. 4A eine erste Winkelausrichtung, bei welcher der Zapfen 72 in einer der mittleren Durchbrüche 74 einrasten kann, so dass sich eine in etwa parallele Ausrichtung der Längserstreckungsrichtungen des Vorderteils 14 und des Sohlenteils 12 ergeben kann. Für die Großzehe des solchermaßen auf der Orthese 10 stehenden Fußes ergibt sich dabei ein mittlerer Korrekturwinkel.

Weiterhin zeigt die Fig. 4B eine zweite Winkelausrichtung, bei welcher der Zapfen 72 beispielsweise in den linken der vier nebeneinander befindlichen Durchbrüche 74 einrasten kann, so dass sich eine deutlich in laterale Richtung orientierte Ausrichtung für die Großzehe ergibt. Eine solche Ausrichtung bzw. Winkelstellung entspricht einem typischen Erscheinungsbild eines Hallux Valgus, bei dem die Großzehe des linken Fußes typischerweise in deutlicher Ausprägung in laterale Richtung gekrümmt ist. Eine Winkelstellung des Vorderteils 14 der Orthese 10 gemäß Fig. 4B kann somit für eine anfängliche Korrektur geeignet sein, bei der es noch nicht sinnvoll ist, die Großzehe in stärkerem Ausmaß in eine mediale Richtung zu verformen, da dies mit hoher Wahrscheinlichkeit zu deutlichen Beschwerden beim betreffenden Patienten führen würde.

Schließlich zeigt die Fig. 4C eine dritte Winkelausrichtung, bei welcher der Zapfen 72 beispielsweise in den rechten der vier nebeneinander befindlichen Durchbrüche 74 einrasten kann, so dass sich eine deutlich in mediale Richtung orientierte Ausrichtung für die Großzehe ergibt. Eine solche Ausrichtung bzw. Winkelstellung entspricht entweder einer nur schwachen Ausprägung eines Hallux Valgus und/oder einem bereits deutlich fortgeschrittenen Therapieerfolg, bei dem die Großzehe des linken Fußes nur noch in geringem Ausmaß in laterale Richtung gekrümmt ist. Eine Winkelstellung des Vorderteils 14 der Orthese 10 gemäß Fig. 4C kann somit für eine deutlich fortgeschrittene Korrektur geeignet sein, bei der die Großzehe in stärkerem Ausmaß in eine mediale Richtung verformt werden kann, wobei dies kaum noch zu Beschwerden beim betreffenden Patienten führen dürfte.

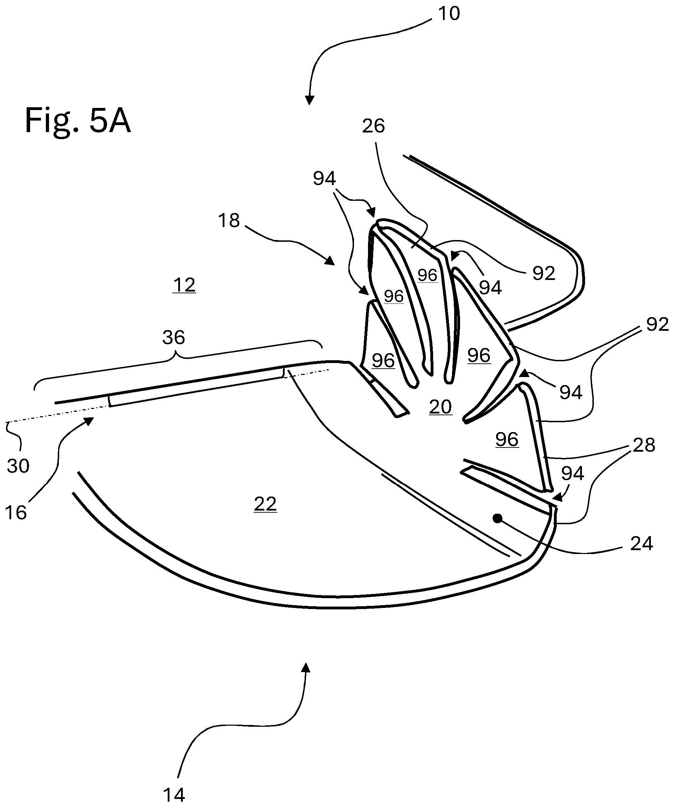

Weiterhin zeigen die Figuren 5A, 5B und 5C in verschiedenen schematischen und perspektivischen Detailansichten eine weitere Ausführungsvariante der erfindungsgemäßen Orthese 10, bei welcher definierte Bereiche des Vorderteiles 14 durch entsprechende Gestaltung flexibel an eine Großzehe 90 (nicht gezeigt, vgl. hierzu aber Fig. 6) eines Benutzers anpassbar sind. Gezeigt sind jeweils Teilansichten einer Orthese 10, die für einen linken Fuß des Benutzers bestimmt ist. Hierbei zeigt die Fig. 5A eine Teilansicht von schräg vorne und oben auf einen vorderen Abschnitt des Sohlenteils 12 mitsamt seinem vorderseitigen Gelenkabschnitt 16 und dem daran gelenkig angeordneten Vorderteil 14. Die Fig. 5B zeigt eine weitere Teilansicht von leicht schräg oberhalb des flachen unteren Abschnittes 22 des Vorderteils 14, so dass der Betrachter auf eine der Großzeh des Benutzers (nicht gezeigt) zugewandten Innenseite der flexiblen Stützwand 20 blickt. Die Fig. 5C zeigt zudem eine Teilansicht von leicht schräg oberhalb des Sohlenteils 12, wobei der Betrachter hier auf eine vom Großzeh des Benutzers (nicht gezeigt) abgewandte und dem benachbarten zweiten Zeh (ebenfalls nicht gezeigt) zugewandte Außenseite der flexiblen Stützwand 20 blickt.

Die in den Figuren 5A, 5B und 5C veranschaulichte weitere Variante der Fuß-Orthese 10 unterscheidet sich in ihren grundlegenden Funktionalitäten nicht von den zuvor erläuterten Varianten gemäß Figuren 1A bis 4C, da hier wie dort der gelenkig an der vorderen Schmalseite des Sohlenteils 12 angeordnete Vorderteil 14 zur Aufnahme der Großzehe (hier: der Großzehe des linken menschlichen Fußes) ausgestaltet ist, wobei die gelenkige Verbindung zwischen den beiden Teilen 12 und 14 über das Verbindungsscharnier 36 erfolgt.

Hinsichtlich der Wirkungsweise des Scharniergelenks 36 ergeben sich keine wesentlichen Unterschiede zum oben bereits Gesagten, so dass auch die in den Figuren 2A bis 2C verdeutlichte Schwenkachse 30 (vgl. Fig. 5C und Fig. 5A) grundsätzlich dieselbe sein kann wie dort. Das Scharniergelenk 36 baut allerdings deutlich schlanker als bei der Ausführungsvariante, wie sie in den Figuren 2A bis 4C in verschiedenen Ansichten gezeigt ist. Die Übergänge von den flachen Ober- und Unterseiten des Sohlenteils 12 zum Scharniergelenk 36 sind bei der in den Figuren 5A bis 5C gezeigten dritten Variante der Orthese 10 weicher und fließender gestaltet, so dass dort keine Stufe gebildet ist. Gleiches gilt für den Übergang vom flachen unteren Abschnitt 22 des Vorderteils 14 zum Scharniergelenk 36, so dass dort ebenfalls keine Stufe gebildet ist. Wie dies insbesondere die Fig. 5C erkennen lässt, kann dasselbe für die gegenüberliegende Unterseite des Abschnittes 22 gelten, die ebenfalls stufenlos in den leicht verdickten Gelenkabschnitt 16 mit dem Scharniergelenk 36 übergeht.

Wiederum weist auch bei der hier beschriebenen weiteren Ausführungsvariante der Orthese 10 gemäß Figuren 5A bis 5C der mittels des um die Gelenkachse 30 schwenkbeweglichen Scharniergelenks 36 an der vorderen Stirnseite des Sohlenteils 12 angeordnete Vorderteil 14 in etwa die Größe und Formgebung auf, dass darauf die Großzehe des auf dem Sohlenteil 12 befindlichen (linken) Fußes (nicht gezeigt) aufliegen kann. Das schlank und weitgehend stufenlos bauende Scharniergelenk 36 des Gelenkabschnittes 16 befindet sich dabei in etwa in einem Bereich unterhalb und vorderhalb eines Zehengrundgelenks (hier ebenfalls nicht gezeigt), so dass ein Abknicken der Großzehe im Gelenk zwischen dem Mittelfußknochen und dem Zehenknochen der Großzehe (vgl. hierzu Fig. 6) durch eine entsprechende Schwenkbewegung im darunter und davor liegenden Scharniergelenk 36 ermöglicht ist (vgl. hierzu etwa Fig. 2C).

Wie es bereits unter Bezugnahme auf die Fig. 2B weiter oben erläutert wurde, ist der über das Scharniergelenk 36 des Gelenkabschnittes 16 stirnseitig am Sohlenteil 12 gelenkig verankerte Vorderteil 14 im Querschnitt L-förmig gestaltet, da der vertikale Stützabschnitt 18 in Gestalt der in etwa vertikal geformten Stützwand 20 gegenüber dem flachen unteren Abschnitt 22 des Vorderteils 14, der am Scharniergelenk 36 ansetzt, nach oben aufragt und die Großzehe einseitig anliegen lässt und dadurch abstützt.

Der Vorderteil 14 der dritten Ausführungsvariante gemäß Figuren 5A bis 5C unterscheidet sich hinsichtlich seiner Gestaltung in wichtigen Details vom Vorderteil gemäß den ersten und zweiten Varianten. Auch bei der dargestellten linken Orthese 10 für den linken Fuß, wie sie jeweils in den Figuren 5A, 5B und 5C gezeigt ist, befindet sich der durch die Stützwand 20 gebildete Stützabschnitt 18 am linken Rand des Vorderteils 14 und stützt somit die mediale Seite der linken Großzehe, d.h. die beim zu behandelnden Beschwerdebild des Hallux Valgus zur linken Körperaußenseite strebende Seite der Großzehe, an die sich die weiteren Zehen des linken Fußes anschließen.

Wie schon weiter oben erläutert, ist die Beweglichkeit des Vorderteils 14 im Gelenkabschnitt 16 durch das Scharniergelenk 36 mit seiner Schwenkachse 30 gewährleistet. Die Schwenkachse 30 liegt in etwa quer zur Längsmittelachse 32 der Fuß-Orthese 10 und in etwa parallel zur flachen Oberfläche 34 des Sohlenteils 12 (vgl. hierzu die Figuren 2B und 2C). Wie ebenfalls weiter oben schon erläutert, kann die Schwenkachse 30, die durch das Scharniergelenk 36 verläuft und dessen Beweglichkeit definiert, vorzugsweise leicht zur Horizontalen abgewinkelt sein, und zwar typischerweise in einem Winkel von etwa fünf Grad. Diese leicht abgewinkelte Ausrichtung der Schwenkachse 30 lässt sich allerdings in den Figuren 5A bis 5C nicht deutlich erkennen. Die Aufgabe und Wirkungsweise dieser Ausrichtung des Vorderteils 14 ist dieselbe wie bereits oben erläutert.

Auch die anhand der Detaildarstellung der Fig. 3 ermöglichte Verstellbarkeit des Vorderteils 14 kann bei der in den Figuren 5A bis 5C erläuterten dritten Variante der Orthese 10 vorgesehen sein, ist aber dort nicht zeichnerisch verdeutlicht. Eine im Ergebnis gleichwertige Verstellbarkeit des Vorderteils 14 kann bspw. auch dadurch realisiert sein, dass verschieden gestaltete Vorderteile 14 durch Öffnen des Gelenkabschnittes 16 gegeneinander ausgetauscht werden können, so dass für unterschiedliche Anatomien, Gelenkformen, Fuß- und/oder Zehenkonturen etc. die jeweils passenden Einzelteile miteinander kombiniert werden können.

Wie es insbesondere die schematische und perspektivische Ansicht von schräg vorne auf die Orthese 10 der Fig. 5A erkennen lässt, kann die segmentierte und dadurch flexibel nachgiebige Stützwand 20 vorzugsweise in einem sanften Rundungsradius 24 vom flachen unteren Abschnitt 22 des Vorderteils 14 übergehen und nach oben hin ebenfalls in sanfter Rundung 26 auslaufen (vgl. hierzu auch die Fig. 1C). Diese obere Rundung 26 der Stützwand 20 kann diese vorzugsweise leicht nach innen zur Großzehe hin ziehen, so dass deren Konturen an ihrer medialen Seite besser gefolgt ist und die Großzehe vom Stützabschnitt 18 teilweise umfangen und abgestützt ist.

Eine äußere umfangsseitige Kante 92 des Stützabschnittes 18 bzw. der Stützwand 20, die nach vorne hin auch die vordere stirnseitige Kante 28 bildet (vgl. Fig. 1C), kann in der gezeigten Weise eine halbrundförmige Kontur haben, so dass die in vertikale Richtung vom in etwa horizontal ausgerichteten flachen unteren Abschnitt 22 aufragende Stützwand 20 die insbesondere in den Figuren 5B und 5C erkennbare kreissegmentförmige Gestalt aufweist, welche die ungefähr halbrundförmige Kontur bildet.

Wie es die Figuren 5A bis 5C deutlich erkennen lassen, ist die vertikale Stützwand 20 segmentiert, und zwar durch strahlenförmig oder radial nach außen in Richtung zur äußeren umfangsseitigen Kante 92 verlaufende Schlitze 94, welche sich innenseitig nicht treffen, sondern nur bis in eine Tiefe der habrundförmig konturierten vertikalen Stützwand 20 reichen, die einem Maß von etwa zwei Dritteln ihres Radius entsprechen kann. Im gezeigten Ausführungsbeispiel ist die Stützwand 20 durch insgesamt sechs solche Schlitze 94 segmentiert, die allesamt in etwa dieselbe Länge aufweisen, wodurch insgesamt fünf ähnlich konturierte und im Groben ähnlich dimensionierte Zungen 96 gebildet sind, die durch die Schlitze 94 voneinander separiert sind und dadurch unabhängig voneinander elastisch ausweichen und zurückfedern können.

Durch Auswahl eines geeigneten Werkstoffes für den Vorderteil 14 weisen die Zungen 96, die in ihrem Verbindungsbereich schmaler sind als an ihren jeweiligen äußeren umfangsseitigen Kanten 92, eine gewisse Elastizität und Nachgiebigkeit auf, so dass sie sich leichter an einen dort flächig anliegenden Großzeh anpassen können. Ein Teil der gesamten flächigen Kontur der Stützwand 20 ist dadurch flexibel verformbar, wodurch der Tragekomfort für den Benutzer erheblich verbessert ist.

Ergänzend sei in diesem Zusammenhang darauf hingewiesen, dass sich die in den Figuren 5A bis 5C beschriebene Flexibilität des Stützwand 20 des Stützabschnittes 18 auch durch andere Maßnahmen erreichen ließe, bspw. durch ein Mehrkomponenten-Spritzgießverfahren, bei dem anstelle der in den Figuren 5A bis 5C gezeigten Schlitze 94 gezielte Dünnstellen mit leicht elastisch dehnbaren Materialeigenschaften vorgesehen sein könnten. Wenn demgegenüber die Zungen 96 mit etwas steiferem Verformungsverhalten noch immer über ein gewisses Maß an Flexibilität verfügen, kann sich eine ähnliche Anpassbarkeit an die Anatomie der jeweiligen Großzehe eines Trägers der Orthese 10 ergeben.

Ein vergleichbares Verformungsverhalten kann sich zudem durch metallene Inlays ergeben, die von Kunststoffmaterial umspritzt sind. Hierbei könnten etwa flache metallene Inlays aus Federblechstreifen im einem elastischen Kunststoffmaterial eingebettet sein, bspw. in einem Elastomermaterial, womit sich in vergleichbarer Weise das beschriebene Verformungsverhalten realisieren ließe.

Abschließend soll die schematische Draufsicht der Fig. 6 einige anatomische Zusammenhänge und die Positionierung der Fußknochen eines auf einer rechten Fuß-Orthese 10 stehenden rechten Fußes in Bezug auf die verschiedenen Bereiche der Orthese 10 verdeutlichen. Diese findet sich dort in unterbrochener Linierung eingezeichnet, während ein Fußskelett 78 mit den hier interessierenden Bereichen in Umrisslinien eingezeichnet ist.

Die Fußwurzelknochen in der Fußwurzel 80 sollen hier nicht weiter interessieren, zumal sie dem Fachmann bekannt sind. Diese Fußwurzelknochen umfassen das Fersen-, das Sprung- und das Kahnbein, außerdem das Würfelbein und die ersten bis dritten Keilbeine, was in Fig. 6 mit einer übergreifenden Klammer links vom Fußskelett 78 verdeutlicht ist, welche die gesamte Fußwurzel 80 bezeichnet.

Der Vorfuß 82 schließt sich in Richtung der Zehen daran an und ist ebenfalls mit einer übergreifenden Klammer links vom Fußskelett 78 verdeutlicht. Der Vorfuß 82 umfasst die ersten bis fünften Mittelfußknochen, wobei im vorliegenden Zusammenhang der erste und größte Mittelfußknochen 84 interessiert, da sich im Gelenk zwischen dem ersten Keilbein der Fußwurzel 80 und dem ersten Mittelfußknochen 84 die hier interessierende und mittels der Orthese 10 zu korrigierende Valgusstellung im Großzehengrundgelenk 86 ergeben kann. Dieses Großzehengrundgelenk 86 ist in der Fig. 6 durch eine Umkreisung mit starker punktierter Linierung hervorgehoben und mit der entsprechenden Bezugsziffer gekennzeichnet.

Die weiter nach vorne reichenden Zehenknochen 88 sollen ebenfalls nicht näher interessieren, lediglich die beiden Knochen der Großzehe 90, die sich im Bereich des Vorderteils 14 der Orthese 10 befinden, so dass dort die ungehinderte Beweglichkeit in Entsprechung zur Verschwenkbarkeit des Vorderteils 14 um den Gelenkabschnitt 16 gegeben ist.

Dagegen lässt die Fig. 6 deutlich erkennen, was mit dem Abstand des Gelenkabschnittes 16 vom Großzehengrundgelenk 86 gemeint ist. So befindet sich der Gelenkabschnitt 16 in etwa unterhalb des Gelenks zwischen den vorderen Zehenknochen 88 und dem ersten Mittelfußknochen 84 der Großzehe 90, während das Großzehengrundgelenk 86 mit deutlichem Abstand dazu im vorderen linken Randbereich des Sohlenteils 12 angeordnet ist. Bei der oben anhand der Figuren 1A bis 4C beschriebenen Korrektur der Hallux-Valgus-Fehlstellung mittels der Orthese 10 wird die Hebelwirkung genutzt, die über die Einbettung der Großzehe im Vorderteil 14 und durch ihre Abstützung am Stützabschnitt 18 auf das Großzehengrundgelenk 86 zurückwirkt.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

10 Orthese, Fuß-Orthese
12 Sohlenteil
14 Vorderteil
16 Gelenkabschnitt
18 Stützabschnitt, vertikaler Stützabschnitt
20 Stützwand, vertikale Stützwand
22 unterer Abschnitt, flacher unterer Abschnitt (des Vorderteils)
24 Rundung, Rundungsradius, gerundeter Radius am Übergang zur Stützwand
26 oberer Rundungsradius der Stützwand
28 vordere Kante, vordere stirnseitige Kante (Stützwand)
30 Gelenkachse, Schwenkachse
32 Längsmittelachse (Fuß-Orthese, Sohlenteil)
34 Oberfläche (Sohlenteil)
36 Scharnier, Scharniergelenk
38 Neigungswinkel, Neigungswinkel der Gelenkachse (gegenüber der Oberfläche des Sohlenteils)
40 mediale Längsseite
42 Auswölbung
44 Hülsenabschnitt, äußerer Hülsenabschnitt
46 Hülsenabschnitt, mittlerer Hülsenabschnitt
48 Hülsenabschnitt, äußerer Hülsenabschnitt
50 Gelenkbolzen
52 Verbindungsstück
54 Bolzenkopf
56 Grundseite (des halbkreisförmigen Verbindungsstückes)
58 Außenmantel (mittlerer Hülsenabschnitt)
60 Vorderseite, halbkreisförmige Vorderseite (des Verbindungsstückes)
62 untere Stufe (mit größerem Radius)
64 obere Stufe (mit kleinerem Radius)
66 Rückseite (des Vorderteils)
68 Aussparung, halbkreisförmige Aussparung (des Vorderteils)
70 Aussparung, muldenförmige Aussparung (des Vorderteils)
72 Zapfen
74 Durchbruch
76 Längsmittelachse (Fuß-Orthese, Vorderteil)
78 Fußskelett
80 Fußwurzel
82 Vorfuß
84 erster Mittelfußknochen, großer Mittelfußknochen
86 Großzehengrundgelenk, Zehengrundgelenk der Großzehe
88 Zehenknochen
90 Großzehe
92 äußere umfangsseitige Kante
94 Schlitz, strahlenförmiger Schlitz, radialer Schlitz
96 Zunge

## Patentansprüche

1. Orthese (10) zur Korrektur und/oder zur Behandlung von Fußfehlstellungen, insbesondere zur Behandlung von Hallux-Valgus, mit einem Sohlenteil (12) zur Aufnahme zumindest eines Teils eines darauf stehenden und sich darauf abstützenden menschlichen Fußes,
- mit einem für die Aufnahme und Abstützung zumindest einer Unterseite einer Großzehe des auf dem Sohlenteil (12) stehenden Fußes sowie zumindest von medialen Teilbereichen der Großzehe (90) vorgesehenen Vorderteil (14),
- welcher Vorderteil (14) über einen Gelenkabschnitt (16) gelenkig mit dem Sohlenteil (12) verbunden ist,
- welcher Gelenkabschnitt (16) sich in einem Bereich unterhalb und vorderhalb eines Zehengrundgelenks (86) der Großzehe (90) bei auf dem Sohlenteil (12) stehendem Fuß befindet.

2. Orthese (10) nach Anspruch 1, bei welcher der gelenkig mit dem Sohlenteil (12) verbundene Vorderteil (14) einen Stützabschnitt (18) zur Abstützung und Aufnahme der Unterseite der Großzehe sowie von medialen Teilbereichen der Großzehe umfasst, wobei der Stützabschnitt (18) in etwa vertikal zu einem flachen unteren Abschnitt (22) des Vorderteils (14) ausgerichtet ist.

3. Orthese (10) nach Anspruch 2, bei welcher der Stützabschnitt (18) des Vorderteils (14) zumindest abschnittsweise einen in etwa L-förmigen Querschnitt aufweist und insbesondere die Großzehe (90) zumindest in medialer Richtung abstützt.

4. Orthese (10) nach einem der Ansprüche 2 oder 3, bei welcher die Schenkel des L-förmigen vorderen Stützabschnittes (18) über einen Verbindungsabschnitt mit einem Rundungsradius (24) verbunden sind, der in etwa einer Kontur der Großzehe (90) folgen kann und diese dort zumindest teilweise einbettet oder zumindest teilflächig abstützt.

5. Orthese (10) nach einem der Ansprüche 2 bis 4, bei welcher die in etwa vertikal orientierte Stützwand (20) des Stützabschnittes (18) zumindest abschnittsweise elastisch verformbar ist, wobei insbesondere eine segmentartige Unterteilung der Stützwand (20) eine Anpassbarkeit an eine Anatomie der Großzehe (90) und/oder des Fußes ermöglicht.

6. Orthese (10) nach Anspruch 5, bei welcher die Stützwand (20) durch mehrere strahlenförmig verlaufende Schlitze (94) segmentiert und/oder in unabhängig voneinander elastisch verformbare Zungen (96) unterteilt ist.

7. Orthese (10) nach einem der Ansprüche 1 bis 6, bei welcher der Gelenkabschnitt (16) durch ein Scharniergelenk (36) gebildet ist, welches sich in etwa unterhalb des Zehengrundgelenks der Großzehe bei auf dem Sohlenteil (12) stehendem Fuß befindet.

8. Orthese (10) nach einem der Ansprüche 1 bis 6, bei welcher der Gelenkabschnitt (16) durch ein Filmscharnier o. dgl. Scharnier gebildet ist, welches sich insbesondere in etwa unterhalb des Zehengrundgelenks der Großzehe bei auf dem Sohlenteil (12) stehendem Fuß befindet.

9. Orthese (10) nach einem der Ansprüche 1 bis 8, bei der eine Schwenk- oder Gelenkachse (30) des Scharniergelenks (36) oder des Filmscharniers gegenüber einer in etwa horizontalen Auflageebene, die ungefähr einer Trennebene zwischen einer Oberseite der Sohlenteils (12) und einer plantaren Unterseite des Fußes entspricht, leicht angewinkelt ist.

10. Orthese (10) nach einem der Ansprüche 7 bis 9, bei der eine Schwenk- oder Gelenkachse (30) des Scharniergelenks (36) oder des Filmscharniers in einem Winkel zwischen etwa 1,5° und etwa 8° in mediale Richtung des betreffenden Fußes, der auf der Orthese (10) steht, nach unten gegenüber der in etwa horizontalen Auflageebene, die ungefähr einer Trennebene zwischen einer Oberseite der Sohlenteils (12) und einer plantaren Unterseite des Fußes entspricht, abgewinkelt ist, so dass die Schwenk- oder Gelenkachse (30) leicht in laterale Richtung des Fußes leicht ansteigt.

11. Orthese (10) nach einem der Ansprüche 7 bis 10, bei der die Schwenk- oder Gelenkachse (30) des Scharniergelenks (36) oder des Filmscharniers in einem Winkel zwischen etwa 3,5° und etwa 6,5° in mediale Richtung des betreffenden Fußes, der auf der Orthese (10) steht, nach unten gegenüber der in etwa horizontalen Auflageebene, die ungefähr einer Trennebene zwischen einer Oberseite der Sohlenteils (12) und einer plantaren Unterseite des Fußes entspricht, abgewinkelt ist, wobei der Winkel vorzugsweise in etwa 5° betragen kann.

12. Orthese (10) nach einem der Ansprüche 1 bis 11, bei welcher die Schwenk- oder Gelenkachse (30) des Scharniergelenks (36) oder des Filmscharniers in etwa rechtwinkelig zu einer Längsmittelachse (32) des Sohlenteils (12) ausgerichtet ist.

13. Orthese (10) nach einem der Ansprüche 1 bis 12, bei welcher eine Längsmittelachse (76) des Vorderteils (14) gegenüber der Längsmittelachse (32) des Sohlenteils (12) in spitzem Winkel leicht angewinkelt ist.

14. Orthese (10) nach einem der Ansprüche 1 bis 13, bei welcher der Vorderteil (14) mit dem Stützabschnitt (18) vom Sohlenteil (12) trennbar und dort anfügbar ist.

15. Orthese (10) nach Anspruch 14, bei dem der Vorderteil (14) mit dem Stützabschnitt (18) mittels einer Rastverbindung mit dem Sohlenteil (12) verbindbar ist.
